# EUROPEAN PATENT APPLICATION

(11) **EP 3 101 134 A1**
(43) Date of publication of application: **07.12.2016**
(21) Application number: 15305861.5
(22) Date of filing: 05.06.2015
(51) Int. Cl.: C12N 15/62, C12N 15/78, C07K 14/025

(54) **THERAPEUTIC VACCINE FOR TREATING OR PREVENTING MERKEL CELL POLYOMA VIRUS-ASSOCIATED TUMORS**

(71) Applicant: Apcure SAS, 69003 Lyon (FR); Altrabio, 69007 Lyon (FR)
(72) Inventor: BUFFAT, Laurent, 69003 LYON (FR); PIERINI, Roberto, 69008 LYON (FR)
(74) Representative: Sarlin, Laure V.

(57) **Abstract**

The invention relates to a therapeutic vaccine useful against tumors exhibiting as signature the Large T antigen (LT) of the Merkel Cell PolyomaVirus (MCPyV). Additionally, the therapeutic vaccine may be also useful for preventing tumors in healthy individuals infected with MCPyV. The therapeutic vaccine involves a type-3 secretion system (T3SS) bacterial vector able to deliver a polypeptide comprising LT epitopes to antigen presenting cells (APCs), such as a truncated form of LT. The invention also relates to a fusion protein comprising a truncated form of LT.

SEQUENCE LISTING

<110> APCURE SAS
      ALTRABIO

<120> Therapeutic Vaccine for Treating or preventing Merkel cell Polyoma
Virus-associated tumors

<130> 1H722510EP1LSA

<160> 26

<170> BiSSAP 1.3

<210> 1
<211> 469
<212> PRT
<213> Merkel cell polyomavirus

<220>
<223> Truncated Large T Antigen position 1 to 469

<400> 1
Met Asp Leu Val Leu Asn Arg Lys Glu Arg Glu Ala Leu Cys Lys Leu
1               5                   10                  15
Leu Glu Ile Ala Pro Asn Cys Tyr Gly Asn Ile Pro Leu Met Lys Ala
            20                  25                  30
Ala Phe Lys Arg Ser Cys Leu Lys His His Pro Asp Lys Gly Gly Asn
        35                  40                  45
Pro Val Ile Met Met Glu Leu Asn Thr Leu Trp Ser Lys Phe Gln Gln
    50                  55                  60
Asn Ile His Lys Leu Arg Ser Asp Phe Ser Met Phe Asp Glu Val Asp
65                  70                  75                  80
Glu Ala Pro Ile Tyr Gly Thr Thr Lys Phe Lys Glu Trp Trp Arg Ser
                85                  90                  95
Gly Gly Phe Ser Phe Gly Lys Ala Tyr Glu Tyr Gly Pro Asn Pro His
            100                 105                 110
Gly Thr Asn Ser Arg Ser Arg Lys Pro Ser Ser Asn Ala Ser Arg Gly
        115                 120                 125
Ala Pro Ser Gly Ser Ser Pro Pro His Ser Gln Ser Ser Ser Ser Gly
    130                 135                 140
Tyr Gly Ser Phe Ser Ala Ser Gln Ala Ser Asp Ser Gln Ser Arg Gly
145                 150                 155                 160
Pro Asp Ile Pro Pro Glu His His Glu Glu Pro Thr Ser Ser Ser Gly
                165                 170                 175
Ser Ser Ser Arg Glu Glu Thr Thr Asn Ser Gly Arg Glu Ser Ser Thr
            180                 185                 190
Pro Asn Gly Thr Ser Val Pro Arg Asn Ser Ser Arg Thr Asp Gly Thr
        195                 200                 205
Trp Glu Asp Leu Phe Cys Asp Glu Ser Leu Ser Ser Pro Glu Pro Pro
    210                 215                 220
Ser Ser Ser Glu Glu Pro Glu Glu Pro Pro Ser Ser Arg Ser Ser Pro
225                 230                 235                 240
Arg Gln Pro Pro Ser Ser Ser Ala Glu Glu Ala Ser Ser Ser Gln Phe
                245                 250                 255
Thr Asp Glu Glu Tyr Arg Ser Ser Ser Phe Thr Thr Pro Lys Thr Pro
            260                 265                 270
Pro Pro Phe Ser Arg Lys Arg Lys Phe Gly Gly Ser Arg Ser Ser Ala
        275                 280                 285
Ser Ser Ala Ser Ser Ala Ser Phe Thr Ser Thr Pro Pro Lys Pro Lys
    290                 295                 300
Lys Asn Arg Glu Thr Pro Val Pro Thr Asp Phe Pro Ile Asp Leu Ser
305                 310                 315                 320

Asp Tyr Leu Ser His Ala Val Tyr Ser Asn Lys Thr Val Ser Cys Phe
                325                 330                 335
Ala Ile Tyr Thr Thr Ser Asp Lys Ala Ile Glu Leu Tyr Asp Lys Ile
            340                 345                 350
Glu Lys Phe Lys Val Asp Phe Lys Ser Arg His Ala Cys Glu Leu Gly
        355                 360                 365
Cys Ile Leu Leu Phe Ile Thr Leu Ser Lys His Arg Val Ser Ala Ile
    370                 375                 380
Lys Asn Phe Cys Ser Thr Phe Cys Thr Ile Ser Phe Leu Ile Cys Lys
385                 390                 395                 400
Gly Val Asn Lys Met Pro Glu Met Tyr Asn Asn Leu Cys Lys Pro Pro
                405                 410                 415
Tyr Lys Leu Leu Gln Glu Asn Lys Pro Leu Leu Asn Tyr Glu Phe Gln
            420                 425                 430
Glu Lys Glu Lys Glu Ala Ser Cys Asn Trp Asn Leu Val Ala Glu Phe
        435                 440                 445
Ala Cys Glu Tyr Glu Leu Asp Glu Val Arg Asp Ala Gly Asn Asp Gln
    450                 455                 460
Asp Arg Asn Ala Met
465

<210> 2
<211> 129
<212> PRT
<213> Pseudomonas

<220>
<223> N-terminal end of ExoS from position 1 to 129

<400> 2
Met His Ile Gln Ser Leu Gln Gln Ser Pro Ser Phe Ala Val Glu Leu
1               5                   10                  15
His Gln Ala Ala Ser Gly Arg Leu Gly Gln Ile Glu Ala Arg Gln Val
            20                  25                  30
Ala Thr Pro Ser Glu Ala Gln Gln Leu Ala Gln Arg Gln Asp Ala Pro
        35                  40                  45
Lys Gly Glu Gly Leu Leu Ala Arg Leu Gly Ala Ala Leu Val Arg Pro
    50                  55                  60
Phe Val Ala Ile Met Asp Trp Leu Gly Lys Leu Leu Gly Ser His Ala
65                  70                  75                  80
Arg Thr Gly Pro Gln Pro Ser Gln Asp Ala Gln Pro Ala Val Met Ser
                85                  90                  95
Ser Ala Val Val Phe Lys Gln Met Val Leu Gln Gln Ala Leu Pro Met
            100                 105                 110
Thr Leu Lys Gly Leu Asp Lys Ala Ser Glu Leu Ala Thr Leu Thr Pro
        115                 120                 125
Glu

<210> 3
<211> 129
<212> PRT
<213> Pseudomonas

<220>
<223> N-terminal of ExoT from position 1 to 129

<400> 3
Met His Ile Gln Ser Ser Gln Gln Asn Pro Ser Phe Val Ala Glu Leu
1               5                   10                  15
Ser Gln Ala Val Ala Gly Arg Leu Gly Gln Val Glu Ala Arg Gln Val
            20                  25                  30
Ala Thr Pro Arg Glu Ala Gln Gln Leu Ala Gln Arg Gln Glu Ala Pro

35                  40                  45
Lys Gly Glu Gly Leu Leu Ser Arg Leu Gly Ala Ala Leu Ala Arg Pro
    50                  55                  60
Phe Val Ala Ile Ile Glu Trp Leu Gly Lys Leu Leu Gly Ser Arg Ala
65                  70                  75                  80
His Ala Ser Thr Gln Ala Pro Leu Ser Arg Gln Asp Ala Pro Pro Ala
                85                  90                  95
Ala Ser Leu Ser Ala Ala Glu Ile Lys Gln Met Met Leu Gln Lys Ala
            100                 105                 110
Leu Pro Leu Thr Leu Gly Gly Leu Gly Lys Ala Ser Glu Leu Ala Thr
        115                 120                 125
Leu

<210> 4
<211> 13
<212> PRT
<213> Artificial Sequence

<220>
<223> PADRE sequence

<400> 4
Ala Lys Phe Val Ala Ala Trp Thr Leu Lys Ala Ala Ala
1               5                   10

<210> 5
<211> 17
<212> PRT
<213> Influenza A virus

<220>
<223> NP68 peptide 362-378

<400> 5
Gly Val Gln Ile Ala Ser Asn Glu Asn Met Asp Ala Met Glu Ser Ser
1               5                   10                  15
Thr

<210> 6
<211> 8
<212> PRT
<213> Artificial Sequence

<220>
<223> Ovalbumine peptide 257 to 264

<400> 6
Ser Ile Ile Asn Phe Glu Lys Leu
1               5

<210> 7
<211> 774
<212> DNA
<213> Artificial Sequence

<220>
<223> nucleotide sequence encoding the amino acid sequence from
      position 2 to 259 of SEQ ID NO : 1 which has been further mutated
      to position 216 (E216K), for optimized expression in Pseudomonas

<400> 7

gacctggttt tgaatcgcaa ggaacgtgaa gcattgtgta agcttctgga gattgccccg 60

aactgctacg ggaatatccc gctgatgaaa gccgctttca agcgctcgtg cctgaagcac 120

cacccggata aggggggcaa cccggtcatt atgatggagc tgaacacgct ttggtcgaag 180

ttccaacaga acatccacaa gctgcgttcg gacttctcca tgttcgatga ggtggacgaa 240

gccccgatct acggcactac caagttcaaa gagtggtggc gctcgggcgg cttctcgttc 300

ggaaaggcat acgagtatgg gccgaaccct catggcacaa acagccgttc gcgcaaaccg 360

agtagtaacg cttcgcgcgg tgccccgagc ggaagctcgc cgccacatag tcagagctcg 420

tccagcgggt atggctcgtt tagtgcaagc caggcaagtg attcccagag tcggggcccg 480

gatataccgc ccgaacacca cgaggagccc acgtcctcca gcggtagctc gagtcgggaa 540

gaaacgacca acagcggacg cgaatcgagc acgcccaatg gcacctcggt tccccgcaat 600

tcctcgcgca ccgatggtac ttgggaagac ttgttttgcg acgagagttt gtcctccccg 660

gagcctccga gcagtagcga ggaaccggaa gagccaccct cgtcccgttc gagtccgcgg 720

caacctccgt cctcctcggc agaggaagca agtagctcgc aattcaccga cgag 774

<210> 8
<211> 387
<212> DNA
<213> Pseudomonas

<220>
<223> nucleotide sequence encoding the amino acid sequence of SEQ ID NO
      : 2 (ExoS) from position 1 to 129

<400> 8
atgcatattc aatcgcttca gcagagtccg tctttcgccg tcgaattgca ccaggccgcc 60

agtgggcgtt tgggacagat tgaggcccgc caggtcgcca cccccagtga agcgcagcag 120

ttggcccagc gccaggacgc gccgaagggt gaggggctgc tcgctcgcct gggcgcggcg 180

ctcgtgcgtc ccttcgtggc gatcatggac tggctgggca aactgttggg ctcccacgcc 240

cgcaccggcc cgcagcccag tcaggacgcg cagcctgcgg tcatgtcctc ggccgtcgtg 300

ttcaagcaga tggtgctgca gcaggcattg cccatgacct tgaagggact cgacaaggcg 360

agcgagctgg cgaccctgac accggaa 387

<210> 9
<211> 39
<212> DNA
<213> Artificial Sequence

<220>
<223> nucleotide sequence encoding the amino acid sequence of SEQ ID NO
      : 4 (PADRE sequence)

<400> 9
gccaagttcg tcgccgcctg gaccctgaag gccgccgcc 39

<210> 10
<211> 51
<212> DNA
<213> Artificial Sequence

<220>
<223> nucleotide sequence encoding the amino acid sequence of SEQ ID NO
      : 5 (peptide NP68) for optimized expression in Pseudomonas

<400> 10
ggcgtgcaga tcgcctccaa cgaaaatatg gacgcgatgg agtcgagcac c          51


<210> 11
<211> 27
<212> DNA
<213> Artificial Sequence

<220>
<223> PCR primer

<400> 11
acttgggaaa gttttgactg gtggcaa                                     27


<210> 12
<211> 26
<212> DNA
<213> Artificial Sequence

<220>
<223> PCR antisense primer

<400> 12
gggcctcgtc aacctagatg ggaaag                                      26


<210> 13
<211> 817
<212> PRT
<213> Merkel cell polyomavirus

<220>
<223> Native Large T antigen from position 1 to 817

<400> 13
Met Asp Leu Val Leu Asn Arg Lys Glu Arg Glu Ala Leu Cys Lys Leu
1               5                   10                  15
Leu Glu Ile Ala Pro Asn Cys Tyr Gly Asn Ile Pro Leu Met Lys Ala
            20                  25                  30
Ala Phe Lys Arg Ser Cys Leu Lys His His Pro Asp Lys Gly Gly Asn
        35                  40                  45
Pro Val Ile Met Met Glu Leu Asn Thr Leu Trp Ser Lys Phe Gln Gln
    50                  55                  60
Asn Ile His Lys Leu Arg Ser Asp Phe Ser Met Phe Asp Glu Val Asp
65                  70                  75                  80
Glu Ala Pro Ile Tyr Gly Thr Thr Lys Phe Lys Glu Trp Trp Arg Ser
                85                  90                  95
Gly Gly Phe Ser Phe Gly Lys Ala Tyr Glu Tyr Gly Pro Asn Pro His
            100                 105                 110
Gly Thr Asn Ser Arg Ser Arg Lys Pro Ser Ser Asn Ala Ser Arg Gly

115                 120                 125
Ala Pro Ser Gly Ser Ser Pro Pro His Ser Gln Ser Ser Ser Ser Gly
    130                 135                 140
Tyr Gly Ser Phe Ser Ala Ser Gln Ala Ser Asp Ser Gln Ser Arg Gly
145                 150                 155                 160
Pro Asp Ile Pro Pro Glu His His Glu Glu Pro Thr Ser Ser Ser Gly
                165                 170                 175
Ser Ser Ser Arg Glu Glu Thr Thr Asn Ser Gly Arg Glu Ser Ser Thr
            180                 185                 190
Pro Asn Gly Thr Ser Val Pro Arg Asn Ser Ser Arg Thr Asp Gly Thr
        195                 200                 205
Trp Glu Asp Leu Phe Cys Asp Glu Ser Leu Ser Ser Pro Glu Pro Pro
    210                 215                 220
Ser Ser Ser Glu Glu Pro Glu Glu Pro Pro Ser Ser Arg Ser Ser Pro
225                 230                 235                 240
Arg Gln Pro Pro Ser Ser Ser Ala Glu Glu Ala Ser Ser Ser Gln Phe
                245                 250                 255
Thr Asp Glu Glu Tyr Arg Ser Ser Ser Phe Thr Thr Pro Lys Thr Pro
            260                 265                 270
Pro Pro Phe Ser Arg Lys Arg Lys Phe Gly Gly Ser Arg Ser Ser Ala
        275                 280                 285
Ser Ser Ala Ser Ser Ala Ser Phe Thr Ser Thr Pro Pro Lys Pro Lys
    290                 295                 300
Lys Asn Arg Glu Thr Pro Val Pro Thr Asp Phe Pro Ile Asp Leu Ser
305                 310                 315                 320
Asp Tyr Leu Ser His Ala Val Tyr Ser Asn Lys Thr Val Ser Cys Phe
                325                 330                 335
Ala Ile Tyr Thr Thr Ser Asp Lys Ala Ile Glu Leu Tyr Asp Lys Ile
            340                 345                 350
Glu Lys Phe Lys Val Asp Phe Lys Ser Arg His Ala Cys Glu Leu Gly
        355                 360                 365
Cys Ile Leu Leu Phe Ile Thr Leu Ser Lys His Arg Val Ser Ala Ile
    370                 375                 380
Lys Asn Phe Cys Ser Thr Phe Cys Thr Ile Ser Phe Leu Ile Cys Lys
385                 390                 395                 400
Gly Val Asn Lys Met Pro Glu Met Tyr Asn Asn Leu Cys Lys Pro Pro
                405                 410                 415
Tyr Lys Leu Leu Gln Glu Asn Lys Pro Leu Leu Asn Tyr Glu Phe Gln
            420                 425                 430
Glu Lys Glu Lys Glu Ala Ser Cys Asn Trp Asn Leu Val Ala Glu Phe
        435                 440                 445
Ala Cys Glu Tyr Glu Leu Asp Asp His Phe Ile Ile Leu Ala His Tyr
    450                 455                 460
Leu Asp Phe Ala Lys Pro Phe Pro Cys Gln Lys Cys Glu Asn Arg Ser
465                 470                 475                 480
Arg Leu Lys Pro His Lys Ala His Glu Ala His His Ser Asn Ala Lys
                485                 490                 495
Leu Phe Tyr Glu Ser Lys Ser Gln Lys Thr Ile Cys Gln Gln Ala Ala
            500                 505                 510
Asp Thr Val Leu Ala Lys Arg Arg Leu Glu Met Leu Glu Met Thr Arg
        515                 520                 525
Thr Glu Met Leu Cys Lys Lys Phe Lys Lys His Leu Glu Arg Leu Arg
    530                 535                 540
Asp Leu Asp Thr Ile Asp Leu Leu Tyr Tyr Met Gly Gly Val Ala Trp
545                 550                 555                 560
Tyr Cys Cys Leu Phe Glu Glu Phe Glu Lys Lys Leu Gln Lys Ile Ile
                565                 570                 575
Gln Leu Leu Thr Glu Asn Ile Pro Lys Tyr Arg Asn Ile Trp Phe Lys
            580                 585                 590
Gly Pro Ile Asn Ser Gly Lys Thr Ser Phe Ala Ala Ala Leu Ile Asp
        595                 600                 605
Leu Leu Glu Gly Lys Ala Leu Asn Ile Asn Cys Pro Ser Asp Lys Leu
    610                 615                 620

Pro Phe Glu Leu Gly Cys Ala Leu Asp Lys Phe Met Val Val Phe Glu
625                 630                 635                 640
Asp Val Lys Gly Gln Asn Ser Leu Asn Lys Asp Leu Gln Pro Gly Gln
                645                 650                 655
Gly Ile Asn Asn Leu Asp Asn Leu Arg Asp His Leu Asp Gly Ala Val
            660                 665                 670
Ala Val Ser Leu Glu Lys Lys His Val Asn Lys Lys His Gln Ile Phe
        675                 680                 685
Pro Pro Cys Ile Val Thr Ala Asn Asp Tyr Phe Ile Pro Lys Thr Leu
    690                 695                 700
Ile Ala Arg Phe Ser Tyr Thr Leu His Phe Ser Pro Lys Ala Asn Leu
705                 710                 715                 720
Arg Asp Ser Leu Asp Gln Asn Met Glu Ile Arg Lys Arg Arg Ile Leu
                725                 730                 735
Gln Ser Gly Thr Thr Leu Leu Leu Cys Leu Ile Trp Cys Leu Pro Asp
            740                 745                 750
Thr Thr Phe Lys Pro Cys Leu Gln Glu Glu Ile Lys Asn Trp Lys Gln
        755                 760                 765
Ile Leu Gln Ser Glu Ile Ser Tyr Gly Lys Phe Cys Gln Met Ile Glu
    770                 775                 780
Asn Val Glu Ala Gly Gln Asp Pro Leu Leu Asn Ile Leu Ile Glu Glu
785                 790                 795                 800
Glu Gly Pro Glu Glu Thr Glu Glu Thr Gln Asp Ser Gly Thr Phe Ser
                805                 810                 815
Gln

<210> 14
<211> 2448
<212> DNA
<213> Artificial Sequence

<220>
<223> nucleotide sequence encoding the amino acid sequence from
      position 2 to 817 of SEQ ID NO : 13 for optimized expression in
      Pseudomonas

<400> 14
gacctggttt tgaatcgcaa ggaacgtgaa gcattgtgta agcttctgga gattgccccg      60

aactgctacg ggaatatccc gctgatgaaa gccgctttca agcgctcgtg cctgaagcac     120

cacccggata aggggggcaa cccggtcatt atgatggagc tgaacacgct ttggtcgaag     180

ttccaacaga acatccacaa gctgcgttcg gacttctcca tgttcgatga ggtggacgaa     240

gccccgatct acggcactac caagttcaaa gagtggtggc gctcgggcgg cttctcgttc     300

ggaaaggcat acgagtatgg gccgaaccct catggcacaa acagccgttc gcgcaaaccg     360

agtagtaacg cttcgcgcgg tgccccgagc ggaagctcgc cgccacatag tcagagctcg     420

tccagcgggt atggctcgtt tagtgcaagc caggcaagtg attcccagag tcggggcccg     480

gatataccgc ccgaacacca cgaggagccc acgtcctcca gcggtagctc gagtcgggaa     540

gaaacgacca acagcggacg cgaatcgagc acgcccaatg gcacctcggt tccccgcaat     600

tcctcgcgca ccgatggtac ttgggaagac ttgttttgcg acgagagttt gtcctccccg     660

gagcctccga gcagtagcga ggaaccggaa gagccaccct cgtcccgttc gagtccgcgg     720

caacctccgt cctcctcggc agaggaagca agtagctcgc aattcaccga cgaggagtat     780

cgctccagca gctttaccac ccctaaaacc ccgccaccct tcagtcgcaa acgcaagttc 840

ggtggttcgc ggtccagcgc cagttccgcc agctccgcta gctttacatc cactcctcca 900

aagcctaaga agaaccgtga aacccccgtc cccaccgact tcccgattga cctgagcgat 960

tatctgagcc atgccgttta ctcgaacaaa accgtgagct gcttcgccat ctacactacc 1020

agcgacaagg ccatcgagtt gtatgacaag atcgagaagt tcaaggtgga cttcaaaagc 1080

cggcacgcct gcgagctcgg ctgcatcctt ctgttcatca cactgagcaa acaccgcgta 1140

tcggcgatca aaaacttctg cagcacgttc tgcacgatca gcttcctcat ctgcaagggc 1200

gtgaacaaga tgccggagat gtataacaac ctgtgcaaac cgccgtacaa gctcctgcaa 1260

gagaataagc cgctccttaa ctatgagttt caggagaagg agaaagaggc gtcctgcaac 1320

tggaacctgg tggcggagtt tgcgtgcgag tatgaactcg acgaccactt cattatcctc 1380

gcccactacc tcgattttgc taagcctttc ccgtgtcaaa agtgcgagaa tcggagccgt 1440

ctgaagcctc acaaagcgca tgaggcccat cattcgaacg cgaaactgtt ctacgaatcc 1500

aagagccaga agacgatttg ccaacaggcg gccgacacgg tccttgccaa acgtcgcctg 1560

gaaatgttgg agatgacacg gaccgagatg ctgtgcaaga agtttaagaa acacctggaa 1620

cgcctccgcg acttggatac tattgatttg ctctactaca tgggcggcgt agcgtggtac 1680

tgttgcctgt tcgaggagtt cgagaagaag ctccagaaaa tcatacagct gctcacagag 1740

aacataccga aataccgcaa catctggttt aagggcccga tcaattccgg caagaccagc 1800

tttgcggcgg cgctgatcga cctcttggaa ggcaaggcgc ttaatatcaa ctgtccaagc 1860

gataagctgc ccttcgagct gggttgcgcc ctggataaat tcatggtggt tttcgaagat 1920

gtgaagggcc agaacagcct gaataaggac ctgcagcccg ggcagggcat taacaatttg 1980

gacaacctgc gggatcatct tgacggtgct gtggcggtca gcctcgagaa aaagcatgtc 2040

aacaagaagc atcaaatctt cccgccctgc atcgtcaccg ccaatgatta ctttatcccg 2100

aaaaccctga tcgcccgctt ctcctatacg cttcacttct ccccaaaggc taatctgcgc 2160

gacagcctgg accagaatat ggagattcgc aagcgccgga tactgcagtc ggggaccacc 2220

ttgttgctgt gcctgatctg gtgtctgccc gacaccacct tcaaaccatg tctgcaggaa 2280

gagattaaga attggaagca gatcctccag tcggaaatca gctacggcaa gttctgtcag 2340

atgattgaaa acgtcgaggc cggccaggac ccgctgctca acatcctgat cgaggaagaa 2400

ggacccgaag aaaccgaaga gacccaggat tcgggcacgt ttagccag 2448

<210> 15
<211> 186
<212> PRT
<213> Merkel cell polyomavirus

<220>
<223> Native small T antigen from position 1 to 186

<400> 15
Met Asp Leu Val Leu Asn Arg Lys Glu Arg Glu Ala Leu Cys Lys Leu
1               5                   10                  15
Leu Glu Ile Ala Pro Asn Cys Tyr Gly Asn Ile Pro Leu Met Lys Ala
            20                  25                  30
Ala Phe Lys Arg Ser Cys Leu Lys His His Pro Asp Lys Gly Gly Asn
        35                  40                  45
Pro Val Ile Met Met Glu Leu Asn Thr Leu Trp Ser Lys Phe Gln Gln
    50                  55                  60
Asn Ile His Lys Leu Arg Ser Asp Phe Ser Met Phe Asp Glu Val Ser
65                  70                  75                  80
Thr Lys Phe Pro Trp Glu Glu Tyr Gly Thr Leu Lys Asp Tyr Met Gln
                85                  90                  95
Ser Gly Tyr Asn Ala Arg Phe Cys Arg Gly Pro Gly Cys Met Leu Lys
            100                 105                 110
Gln Leu Arg Asp Ser Lys Cys Ala Cys Ile Ser Cys Lys Leu Ser Arg
        115                 120                 125
Gln His Cys Ser Leu Lys Thr Leu Lys Gln Lys Asn Cys Leu Thr Trp
    130                 135                 140
Gly Glu Cys Phe Cys Tyr Gln Cys Phe Ile Leu Trp Phe Gly Phe Pro
145                 150                 155                 160
Pro Thr Trp Glu Ser Phe Asp Trp Trp Gln Lys Thr Leu Glu Glu Thr
                165                 170                 175
Asp Tyr Cys Leu Leu His Leu His Leu Phe
            180                 185

<210> 16
<211> 555
<212> DNA
<213> Artificial Sequence

<220>
<223> nucleotide sequence encoding the amino acid sequence from
      position 2 to 186 of SEQ ID NO : 15 for optimized expression in
      Pseudomonas

<400> 16
gacctggttt tgaatcgcaa ggaacgtgaa gcattgtgta agcttctgga gattgccccg      60

aactgctacg ggaatatccc gctgatgaaa gccgctttca agcgctcgtg cctgaagcac     120

cacccggata aggggggcaa cccggtcatt atgatggagc tgaacacgct ttggtcgaag     180

ttccaacaga acatccacaa gctgcgttcg gacttctcca tgttcgatga ggtgtccacc     240

aagtttccct gggaagagta cggcacactg aaggattaca tgcaatccgg atacaatgcg     300

cggttttgcc gcggccctgg ttgtatgctg aaacagctgc gcgacagcaa atgcgcctgc     360

atcagttgca aactgagccg gcagcattgc agcctcaaaa ccctcaagca gaagaactgc     420

ttgacctggg gcgaatgctt ctgttatcag tgcttcatcc tctggttcgg cttcccccca     480

acttgggaga gcttcgactg gtggcagaag acgctcgagg agaccgacta ttgtttgctc     540

catctgcatc tgttt                                                      555

<210> 17
<211> 318

<212> DNA
<213> Artificial Sequence

<220>
<223> nucleotide sequence encoding the amino acid sequence from
      position 81 to 186 of SEQ ID NO : 15 for optimized expression in
      Pseudomonas

<400> 17
accaagtttc cctgggaaga gtacggcaca ctgaaggatt acatgcaatc cggatacaat      60

gcgcggtttt gccgcggccc tggttgtatg ctgaaacagc tgcgcgacag caaatgcgcc     120

tgcatcagtt gcaaactgag ccggcagcat tgcagcctca aaaccctcaa gcagaagaac      180

tgcttgacct ggggcgaatg cttctgttat cagtgcttca tcctctggtt cggcttcccc     240

ccaacttggg agagcttcga ctggtggcag aagacgctcg aggagaccga ctattgtttg     300

ctccatctgc atctgttt                                                   318


<210> 18
<211> 420
<212> DNA
<213> Artificial Sequence

<220>
<223> nucleotide sequence encoding the amino acid sequence from
      position 2 to 141 of SEQ ID NO : 15 for optimized expression in
      Pseudomonas

<400> 18
gacctggttt tgaatcgcaa ggaacgtgaa gcattgtgta agcttctgga gattgccccg      60

aactgctacg ggaatatccc gctgatgaaa gccgctttca agcgctcgtg cctgaagcac     120

cacccggata aggggggcaa cccggtcatt atgatggagc tgaacacgct ttggtcgaag      180

ttccaacaga acatccacaa gctgcgttcg gacttctcca tgttcgatga ggtgtccacc     240

aagtttccct gggaagagta cggcacactg aaggattaca tgcaatccgg atacaatgcg     300

cggttttgcc gcggccctgg ttgtatgctg aaacagctgc gcgacagcaa atgcgcctgc     360

atcagttgca aactgagccg gcagcattgc agcctcaaaa ccctcaagca gaagaactgc     420


<210> 19
<211> 183
<212> DNA
<213> Artificial Sequence

<220>
<223> nucleotide sequence encoding the amino acid sequence from
      position 81 to 141 of SEQ ID NO : 15 for optimized expression in
      Pseudomonas

<400> 19
accaagtttc cctgggaaga gtacggcaca ctgaaggatt acatgcaatc cggatacaat      60

gcgcggtttt gccgcggccc tggttgtatg ctgaaacagc tgcgcgacag caaatgcgcc     120

tgcatcagtt gcaaactgag ccggcagcat tgcagcctca aaaccctcaa gcagaagaac 180

tgc 183

<210> 20
<211> 387
<212> DNA
<213> Pseudomonas

<220>
<223> nucleotide sequence encoding the amino acid sequence of SEQ ID NO
      : 3 (ExoT) from position 1 to 129

<400> 20
atgcatattc aatcatctca gcagaacccg tctttcgtgg ctgagttgag ccaggccgtg 60

gccgggcgcc tgggacaggt cgaggcccgc caggtggcca ctccccggga ggcgcaacaa 120

ctggcccagc gccaggaagc accgaagggc gagggcctgc tctcccgcct gggggccgcc 180

ctcgcgcgtc ccttcgtggc gatcatcgag tggctgggca aactgctggg gagccgtgcc 240

cacgcctcca cccaggcgcc gctctcccgt caggacgcgc cgcctgccgc cagtctctcg 300

gccgccgaga tcaagcagat gatgctgcaa aaggcactgc ccctgacctt gggcggactt 360

ggcaaggcga gcgagctggc gactttg 387

<210> 21
<211> 717
<212> DNA
<213> Artificial Sequence

<220>
<223> nucleotide sequence encoding for the green fluorescent protein

<400> 21
atggtgagca agggcgagga gctgttcacc ggggtggtgc ccatcctggt cgagctggac 60

ggcgacgtaa acggccacaa gttcagcgtg tccggcgagg gcgagggcga tgccacctac 120

ggcaagctga ccctgaagtt catctgcacc accggcaagc tgcccgtgcc ctggcccacc 180

ctcgtgacca ccctgaccta cggcgtgcag tgcttcagcc gctaccccga ccacatgaag 240

cagcacgact tcttcaagtc cgccatgccc gaaggctacg tccaggagcg caccatcttc 300

ttcaaggacg acggcaacta caagacccgc gccgaggtga agttcgaggg cgacaccctg 360

gtgaaccgca tcgagctgaa gggcatcgac ttcaaggagg acggcaacat cctggggcac 420

aagctggagt acaactacaa cagccacaac gtctatatca tggccgacaa gcagaagaac 480

ggcatcaagg tgaacttcaa gatccgccac aacatcgagg acggcagcgt gcagctcgcc 540

gaccactacc agcagaacac ccccatcggc gacggccccg tgctgctgcc cgacaaccac 600

tacctgagca cccagtccgc cctgagcaaa gaccccaacg agaagcgcga tcacatggtc 660

ctgctggagt tcgtgaccgc cgccgggatc actctcggca tggacgagct gtacaag 717

<210> 22
<211> 642
<212> DNA
<213> Artificial Sequence

<220>
<223> nucleotide sequence encoding the amino acid sequence from
      position 2 to 215 of SEQ ID NO : 1 for optimized expression in
      Pseudomonas

<400> 22
   gacctggttt tgaatcgcaa ggaacgtgaa gcattgtgta agcttctgga gattgccccg      60
   aactgctacg ggaatatccc gctgatgaaa gccgctttca agcgctcgtg cctgaagcac     120
   cacccggata aggggggcaa cccggtcatt atgatggagc tgaacacgct ttggtcgaag     180
   ttccaacaga acatccacaa gctgcgttcg gacttctcca tgttcgatga ggtggacgaa     240
   gccccgatct acggcactac caagttcaaa gagtggtggc gctcgggcgg cttctcgttc     300
   ggaaaggcat acgagtatgg gccgaaccct catggcacaa acagccgttc gcgcaaaccg     360
   agtagtaacg cttcgcgcgg tgccccgagc ggaagctcgc cgccacatag tcagagctcg     420
   tccagcgggt atggctcgtt tagtgcaagc caggcaagtg attcccagag tcggggcccg     480
   gatataccgc ccgaacacca cgaggagccc acgtcctcca gcggtagctc gagtcgggaa     540
   gaaacgacca acagcggacg cgaatcgagc acgcccaatg gcacctcggt tccccgcaat     600
   tcctcgcgca ccgatggtac ttgggaagac ttgttttgcg ac                        642


<210> 23
<211> 774
<212> DNA
<213> Artificial Sequence

<220>
<223> nucleotide sequence encoding the amino acid sequence from
      position 1 to 259 of SEQ ID NO : 1 which has been further mutated
      to position 216 (E216K)

<400> 23
atggaccttg tccttaacag gaaggagcgt gaggctttgt gcaagctgct ggaaatcgct      60

ccaaactgct acggtaacat ccctctgatg aaggctgcct tcaagcgaag ctgtctcaag     120

caccatcctg acaagggtgg aaaccctgtg atcatgatgg agctcaacac cctgtggtca     180

aaattccagc aaaacattca caagctccga tccgacttct ccatgtttga cgaggttgac     240

gaagccccta tatacgggac caccaagttt aaagagtggt ggaggtccgg aggattcagc     300

tttgggaaag catacgagta tgggcccaac ccccacggaa ccaattcccg gtcccggaaa     360

ccttcctcca atgcttccag gggcgcacca agcggttctt cccctccaca ttctcaatct     420

tccagctccg gttatggctc tttctccgca tcacaggcct cagattctca gagcagaggc     480

ccagatattc ctccagaaca tcacgaggag ccaacttcta gtagcgggtc aagtagtaga     540

gaggagacta ccaatagcgg cagagagagc agcacaccaa atggcacaag cgtacctcgc 600

aatagcagcc gcacagatgg cacatgggaa gatttgtttt gtgacaaatc actgtcttct 660

cctgagcccc ccagttcttc tgaggaaccc gaagaacccc catcatcacg cagtagtccc 720

cggcagcccc cctcttcaag tgccgaagaa gccagtagtt ctcagtttac tgat 774

<210> 24
<211> 564
<212> DNA
<213> Foot-and-mouth disease virus

<220>
<223> Nucleotide sequence encoding an IRES

<400> 24
cccccccccc ctaacgttac tggccgaagc cgcttggaat aaggccggtg tgcgtttgtc 60

tatatgttat tttccaccat attgccgtct tttggcaatg tgagggcccg gaaacctggc 120

cctgtcttct tgacgagcat tcctaggggt ctttcccctc tcgccaaagg aatgcaaggt 180

ctgttgaatg tcgtgaagga agcagttcct ctggaagctt cttgaagaca aacaacgtct 240

gtagcgaccc tttgcaggca gcggaacccc ccacctggcg acaggtgcct ctgcggccaa 300

aagccacgtg tataagatac acctgcaaag gcggcacaac cccagtgcca cgttgtgagt 360

tggatagttg tggaaagagt caaatggctc tcctcaagcg tattcaacaa ggggctgaag 420

gatgcccaga aggtacccca ttgtatggga tctgatctgg ggcctcggtg cacatgcttt 480

acatgtgttt agtcgaggtt aaaaaacgtc taggcccccc gaaccacggg gacgtggttt 540

tcctttgaaa aacacgatga taat 564

<210> 25
<211> 235
<212> DNA
<213> Artificial Sequence

<220>
<223> nucleotide sequence of promotor EF1a

<400> 25
cgtgaggctc cgatgcccgt cagtgggcag agcgcacatc gcccacagtc cccgagaagt 60

tggggggagg ggtcggcaat tgaaccggtg cctagagaag gtggcgcggg gtaaactggg 120

aaagtgatgt cgtgtactgg ctccgccttt ttcccgaggg tgggggagaa ccgtatataa 180

gtgcagtagt cgccgtgaac gttctttttc gcaacgggtt tgccgccaga acaca 235

<210> 26
<211> 928
<212> PRT
<213> Homo sapiens

<220>
<223> amino acid sequence of pRb

<400> 26

## Description

The invention relates to a therapeutic vaccine useful against tumors exhibiting as signature a truncated form of the Large T antigen (LT) of the Merkel Cell Polyoma Virus (MCPyV). Additionally, the therapeutic vaccine may also be useful for preventing tumors in healthy individuals infected with MCPyV. The therapeutic vaccine involves a type-3 secretion system (T3SS) bacterial vector able to deliver a polypeptide comprising MCPyV LT epitopes to antigen presenting cells (APCs).

In a general manner, anti-tumor responses generated by individuals are T-cell-mediated. More specifically, tumor-specific CD8 cytotoxic T-cells are involved. However, T-cells cannot recognize and therefore cannot respond to free antigen. T-cells can only "see" an antigen that has been processed and presented by cells via carrier molecules called Major Histocompatibility Complex (MHC) molecules. Naïve T-cells (*i.e*., T-cells that have not been exposed to antigen) can be primed / activated by a specific class of cells designated under the term "Antigen-Presenting Cells" (APCs) among which dendritic cells (DCs) are the most important, with the broadest range of antigen presentation. APCs express MHC class II as well as class I molecules and can therefore stimulate CD4 and CD8 T-cells, respectively.

Over the past 40 years, bacterial vectors for *in vivo* delivery of heterologous antigens into APCs have been developed. Bacterial vectors offer multiple advantages: (1) there are several well-characterized virulence-attenuation mutations; (2) the number, the amount and the *in vivo* location of antigen expression can be regulated; (3) multiple vaccine delivery routes are possible; and (4) they potently stimulate the innate and adaptive immune systems.

Bacteria are involved in a very wide diversity of biotic associations, ranging from biofilms to mutualistic or pathogenic associations with larger host organisms. Protein secretion plays a central role in modulating all of these interactions. In Gram-negative bacteria, six secretion pathways have been reported until now, each of them being essentially specialized in the translocation of a specific range of proteins. One of them, the type-3 secretion system (T3SS), has been more particularly explored to achieve the delivery of antigenic proteins into APCs, as it can deliver proteins across the bacterial and host cell membranes, into the cytosol of host cells.

The structural core element of T3SS is a syringe-like needle complex (also known as injectisome and involving about 30 proteins), which is embedded across the inner and outer bacterial membranes and extends into the extracellular space. In more details, this syringe-like needle complex comprises two essential components: (i) a membrane-anchored base ring and (ii) an extracellular needle (Tseng et al, BMC Biotech. (2009) 9 (suppl 1) : 52). When expressed by the bacteria, T3SS may exist in an active or inactive state, depending on a number of natural switch factors. T3SS is a critical virulence factor used by several animal and plant pathogens, to deliver into the cytosol of host cells *i.e*., a number of exotoxins generically referred to as T3SS exotoxins. T3SS exotoxins are addressed to T3SS thanks to a short signaling sequence. When fusing an antigen of interest to this signaling sequence, the recombinant protein can be engaged in T3SS and delivered to the cytosol of epithelial cells and circulating DCs, upon appropriate administration of the bacterial vector.

Merkel Cell Polyoma Virus (MCPyV) has first been identified in 2008 as responsible for the oncogenesis of Merkel Cell Carcinoma (MCC) (Shuda et al, 2008 ; Feng et al, 2008), a rare but devastating disease of the skin, and is now detected in all MCC cases (Rodig et al, 2012). Cells are primarily infected with MCPyV, the viral genome of which may remain in a non-integrative form for an indefinite period of time. MCC arises from a two-step process, in which the viral genome (i) integrates into the host genome at any stage of infection and (ii) develops LT truncation mutations to prevent autonomous viral genome replication. Failure to truncate LT may lead to DNA damages responses or immune recognition that hinders nascent tumor cell survival.

More recently, the presence of MCPyV has been detected in at least 20% of non-small cell lung cancer (NSCLC) patients (Hashida et al, 2013) and a role of MCPyV in the NSCLC oncogenesis has been suggested (Antoniou et al, 2013). Also, there is an increasing evidence of MCPyV detection in several types of cancer : cutaneous squamous cell carcinoma (Murakami et al, 2011; Dworkin et al, 2009 ; Kassem et al, 2009 ; Scola et al, 2012 ; Karia et al, 2013), chronic lymphocytic leukemia (Cimino et al, 2013 ; Pantulu et al, 2010), cutaneous B- and T-cell lymphoma (Kreuter et al, 2011 ; Duthanh et al, 2013) ; and cervical squamous cell carcinoma and adenocarcinoma (Imajoh et al, 2012).

As already mentioned above, expression of truncated forms of the large T viral antigen (LT) is the signature of MCPyV-associated tumors (Shuda et al, PNAS (2008) 105 (42) : 16272). Indeed, LT is a protein of about 817 amino acids and has on the host cell, both growth-promoting and inhibitory activities (Cheng et al, J. Virol. (2013) 87 (11) : 6118). The N-terminal domain, including the LXCXE motif of the human retinoblastoma protein binding site (RBS), is responsible for growth-promoting activity, while the C-terminal domain (last 100 amino acids) of the protein is responsible for growth-inhibiting activity. Upon truncation mutation, the C-terminal domain may be lost, the N-terminal domain still being expressed.

As already mentioned above, expression of LT truncated forms first requires integration into the cell genome of a viral nucleotide sequence encoding LT, at a location accessible for transcription. Should a mutation event occur, introducing a stop codon downstream the region encoding *i.e*., the RBS (exon 2) and upstream of the region encoding the C-terminal domain (exon 3), then oncogenic LT truncated forms may be expressed.

The oncogenic effect primarily operates upon binding of the LT truncated form to the human retinoblastoma protein (pRb) which is a cellular protein regularly involved in the negative regulation of cell growth. LT-binding inactivates pRb which is therefore unable to suppress tumor growth events.

Zeng et al, Vaccine (2012) 30 (7) : 1322 have shown that in a mouse model, a prophylactic DNA vaccine encoding a truncated form of the large T antigen of the MCPyV generates anti-tumor effects mainly mediated by LT-specific CD4 helper T-cells. This cannot however be considered as a fully promising result toward the making of a vaccine for MCPyV-positive tumors, since there is a general consensus for an effective anti-tumor vaccine to be able to induce a strong, and otherwise predominant CD8+ immune response (*versus* CD4+) against tumoral cells. Indeed, the importance of CD8 T-cells in the control and eradication of viruses has been acknowledged for long. Furthermore, tumor-reactive CD8 cytotoxic T-cells have consistently been found with improved patient outcomes.

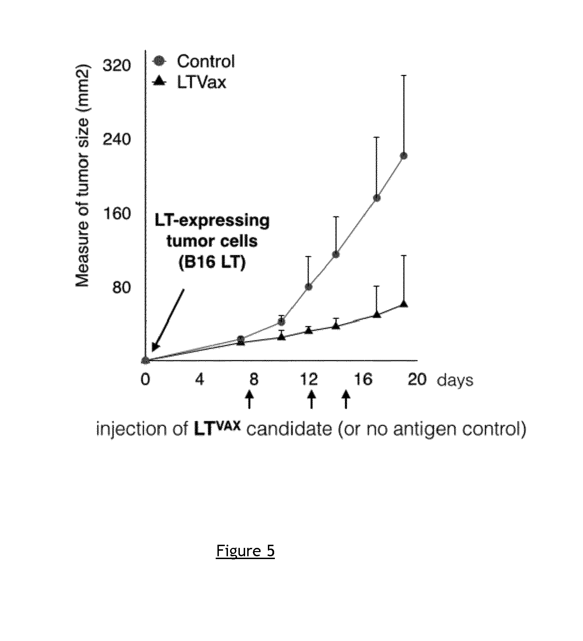

In view of this, Gomez et al, Cell & Bioscience (2012) 2 : 36 have attempted to improve the DNA vaccine of Zeng et al, so that the CD4 T-cell response be switched to a predominant CD8 T-cell response. To this end, they have generated a DNA vaccine encoding a truncated LT antigen tagged to calreticulin (CRT). CRT is a chaperone protein naturally located in the endoplasmic reticulum (ER) where it may participate to correct folding of newly made proteins. Calreticulin has also been shown to associate with peptides delivered into the ER by transporters associated with antigen processing (TAP-1 and TAP-2) and with MHC class I-β2 microglobulin molecules and has further demonstrated the ability to enhance peptide presentation to MHC class I molecules, therefore promoting the induction of antigen-specific CD8 T-cells (Basu et al, J. Exp. Med. (1999) 189 (5) : 797). Consistently, Gomez et al have shown that in a mouse tumor model, a therapeutic DNA vaccine encoding truncated LT tagged to CRT improved mice survival, compared to a DNA vaccine encoding truncated LT alone, which did not perform any better than the negative control (Figure 5 of Gomez et al).

CRT ability to enhance antigen-specific immune response can only be exploited in the context of vaccine technologies based on nucleic acid transfer and subsequent protein neosynthesis. Indeed, neosynthetized proteins enter the ER before degradation into peptides, while this is not the case when proteins are directly delivered to the cells. Indeed, for antigen presentation in the ER, ready-made proteins are first degraded into peptides upon translocation from the cytoplasm into ER. Accordingly, the use of a CRT tag is irrelevant in the context of vaccine technologies based on protein transfer (no DNA or RNA vaccine).

Surprisingly, against the teaching of Gomez et al, it has now been found that an LT-specific CD8 T-cell response can be generated, while delivering LT *via* a vaccine technology based on protein transfer which accordingly does not involve the use of a CRT tag. More specifically, a bacteria! vector able to express LT (in the absence of CRT tag) and to secrete and transfer, preferably translocate LT into mammalian cells *via* a type-3 secretion system (T3SS) was used.

In addition to this, despite the fact that bacterial vectors owning a T3SS are proposed in the art for antigen delivery, not all proteins have the potential to move into T3SS, although fused to appropriate T3SS signaling sequence. This is shown in particular in Radics et al, 2014 which reports that fusion constructs wherein GFP (Green Fluorescent Protein) is fused at the C-termini of otherwise-allowed T3SS substrates, are expressed but not secreted. Indeed, their structure might be too rigid to get unfold for translocation through the T3SS needle. As the tridimensional structure of LT still remains unknown, there is no evidence in the art that LT of MCPyV could be a molecule flexible enough to be efficiently secreted *via* T3SS.

This is the reason why the invention relates to a bacterial vector owning a type 3 secretion system (T3SS bacterial vector), said bacterial vector being able to express, secrete and transfer, preferably translocate, into mammalian cells, a fusion protein which comprises from its N-terminal end to its C-terminal end and fused in frame :
- at least one secretion peptide signal that directs said fusion protein to the type 3 secretion system of the bacterial vector, and
- one truncated form of the Large T antigen (LT) of the Merkel Cell Polyoma Virus (MCPyV), wherein the LT truncated form has an amino acid sequence having at least 80% identity with one of the amino acid sequences shown in SEQ ID NO : 1 which starts with the amino acid in any one of positions 1 to 5 and ends with the amino acid in any one of positions 210 to 469
and, preferably, provided that said fusion protein does not bind to the human retinoblastoma protein.

The invention also relates to a fusion protein which comprises from its N-terminal end to its C-terminal end and fused in frame :
- at least one secretion peptide signal able to direct said fusion protein to the type 3 secretion system of a bacterial vector, when said fusion protein is in a bacteria! vector owning a type 3 secretion system, and
- one truncated form of the Large T (LT) antigen of the Merkel Cell Polyoma Virus (MCPyV), which has an amino acid sequence having at least 80 % identity with one of the amino acid sequences shown in SEQ ID NO : 1 which starts with the amino acid in any one of positions 1 to 5 and ends with the amino acid in any one of positions 210 to 469,
and, preferably, provided that said fusion protein does not bind to the human retinoblastoma protein.

Another object of the invention relates to a bacterial vector of the invention for use in a method of combating MCPyV infection, in particular by promoting a CD8+ immune response against MCPyV-infected cells.

Another object of the invention relates to the bacterial vector of the invention for use as a medicament, preferably for use as a medicament in the treatment of cancer.

The present invention will first be described in general overview. Then, each element will be described in further detail below.

In the following description the standard one letter nucleic acid and the standard one letter amino acid code is used. The standard three letters amino acid code may also be used. All protein descriptions are made from its N-terminal domain to its C-terminal domain.

In practicing the present invention, many conventional techniques in molecular biology and recombinant DNA technology are used. Such techniques are well-known and are explained fully in, for example, Sambrook et al., 2012, Molecular Cloning: A Laboratory Manual, Fourth Edition, Cold Spring Harbor Laboratory Press.

By "vector owning a type 3 secretion system (T3SS vector)" is meant a vehicle having a functional type 3 secretion system capable of delivering proteins from inside of the vehicle into mammalian cells directly or indirectly. In particular, the vector is able to secrete and transfer, preferably translocate proteins into mammalian cells. More preferably, the vector is able to express, secrete and transfer, preferably translocate proteins into mammalian cells.

According to the invention, the vector is preferably a vector of bacterial nature or bacterial origin and is called a bacterial vector.

A bacterial vector of the invention may be a vector of bacterial origin such as particles derived from replication-deficient bacteria, such as bacterial minicells, or may be a vector of bacterial nature such as a bacterium, and in particular a modified bacterium.

Bacterial minicells are small, semi-spherical, bacterial nano-particles that contain all of the components of the parental bacteria, except chromosomes. Without chromosomes, they cannot divide and are non-infectious. Minicells may comprise the type 3 secretion system, thereby allowing the minicells to efficiently deliver proteins into mammalian cells. Bacterial minicells are described by H.A Carleton et al. Nature Communications 4, Article number: 1590 doi:10.1038/ncomms2594.

Preferably, the bacterial vector is a genetically modified bacterium and in particular an attenuated bacterium.

Advantageously, the bacterium vector is a bacterium which is less toxicogenic than the wild-type corresponding bacterium. Advantageously, toxicogenicity can be reduced to an extent that is sufficient to label a bacterial vector as non-toxicogenic. Typically, a bacterial vector is a bacterium that is acknowledged (before clinical use), as being suitable for administration to human beings, and especially to cancer patients.

In one preferred embodiment, the T3SS bacterial vector owning a type 3 secretion system may be a bacterium belonging to any bacterium genus owning a T3SS, for instance to the genus of Gram-negative bacteria. Gram-negative bacteria owning a T3SS are for example *Salmonella, Shigella, Yersinia* and *Pseudomonas*. In a preferred embodiment, the bacterial vector is a bacterium which belongs to the genus of Pseudomonas, in particular to the bacterial species *Pseudomonas aeruginosa* or *Pseudomonas syringae*.

A T3SS bacterial vector according to the invention is typically a T3SS bacterium which derives from a wild-type T3SS bacterium and is significantly less toxicogenic than the corresponding wild-type bacterium. In one embodiment, the bacterial vector according to the invention is unable to express at least one of the products chosen among the *exo*S, *exo*T, *exo*U and *exo*Y gene products and NDK cytotoxin, preferably is unable to express at least the *exo*S, *exo*T and exoU gene products. Advantageously, a T3SS bacterial vector is unable to express at least one, advantageously at least two or three T3SS exotoxins of the wild-type bacterium. Toxicogenicity may be reduced by inactivating at least one T3SS exotoxin genes (T3SS exo genes). Inactivation may be achieved by mutation *i.e.*, conveniently by deletion of whole or part of the gene. In a further improvement, exo genes other than T3SS *exo* genes may also be inactivated.

In a particular embodiment of the invention, the bacterial vector is a bacterium belonging to the *Pseudomonas* genus with reduced toxicogenicity in that it is unable to express and/or secrete at least one, advantageously two T3SS exotoxins including *i.e.*, ExoS, ExoT, ExoU, ExoY and NDK (nucleoside diphosphate kinase) cytotoxin ; and preferably at least the ExoS, ExoT and ExoU exotoxins. Inability to express and/or secrete an *exo* gene product can be achieved by inactivating the *exo* gene, *e.g.,* by deleting whole or part of the *exo* gene. A *Ps. aeruginoso* bacterium unable to express at least one exotoxin (ExoU) is the CHA strain, available in the art (Toussaint et al, Biochem. Biophys. Res. Commun. (1993) 196 : 416). A *Ps. aeruginosa* bacterium unable to express the ExoS, ExoT and ExoU exotoxins is the CHA strain exhibiting additional Δ*exo*S and Δ*exo*T inactivations; such a strain is available in the art under the name CHA-OST or CHAΔST*lox* (Quénée et al, BioTechniques (2005) 38 : 63). The CHA strain may also be available at the Collection Nationale de Culture de Microorganismes (CNCM, Institut Pasteur, Paris) under the name CHA-003 and reference number I-3090 (deposited on September 17, 2003, under the Budapest Treaty by the University Joseph Fourier (Grenoble, FR). For more details, it is possible to refer to WO 2005/049644.

In the following description, the expression "bacterial vector" or "T3SS bacterial vector" "T35S vector" are synonymous.

By "express", it is meant that the bacterial vector is able to translate nucleic sequences, thereby allowing the synthesis of proteins encoded by said nucleic sequences. The protein expression may be checked by any one of the biochemistry technologies well-known in the art, such as Western Blot analysis of lysate of the bacterial vector. The protein expression may also be checked as described in Epaulard O et al, Mol. Ther. (2006) 14 : 656.

By "secrete and transfer into mammalian cells", it is meant that the bacterial vector is able to send proteins having a secretion peptide signal at distance into mammalian cells, more specifically into the cytosol of the mammalian cells. Therefore, said proteins are displaced from a place to another place, said place being separated by membranes. The delivery occurs preferably directly from the bacterial vector into the mammalian cell. In this case, the secretion and the transfer correspond to a translocation. It is also possible that the protein is intermediary delivered in the environment surrounding the bacterial vector and the mammalian cell and after transfered in the mammalian cell (Edgren T et al. PLoS Pathog. 2012;8(5):e1002669. doi: 10.1371/journal.ppat.1002669. Epub 2012 May 10.) The secretion peptide signal is recognized by the complex of the T3SS system. These proteins are then transported via the T3SS system from the interior of the bacterial vector (such as from the cytosol of the bacterial vector), to its exterior and are preferably injected directly into the cytoplasm of the mammalian cells. The T3SS acting in this case like a syringe. Proteins pass through the inner membrane, the periplasmic space and the outer membrane of the bacterial vector, and the membrane of the mammalian cells. The delivery, i.e the secretion and transfer, of proteins can be assessed by the methods described in the example. The secretion of protein may be assessed by a secretion assay. This consists in the detection of the secreted protein in the bacterial supernatant. The detection is done on an acrylamide gel and comassie staining or by Western Blot. An example of a secretion assay is described in Epaulard O et al, Mol. Ther. (2006) 14 : 656.

By "translocate into mammalian cells," it is meant that the secretion and the transfer of proteins into the mammalian cells, such as into the cytosol of the mammalian cells, occur directly, the proteins being directly displace from the bacterial vector to the mammalian cell, through the T3SS system, acting like a syringe. The translocation can be assessed by the methods described in the example or in Le Gouëllec A et al Mol Ther. 2013 May; 21(5):1076-86. doi: 10.1038/mt.2013.41. Epub 2013 Mar 26. PMID: 23531551.

By "mammalian cells", it is meant, any animal, preferably human, eukaryotic cells present in vitro, ex vivo or in vivo in research, diagnostic or therapeutic applications. For example, mammalian cells may be cells of the immune system, in particular antigen presenting cells (APCs) like monocytes, dendritic cells, macrophages, B cells and NK cells. In one preferred embodiment, the mammalian cells are dendritic cells.

By "fusion protein", it is meant a protein artificially created from at least two peptides fragments (or moieties) of different origins, which are fused either directly (generally by a peptide bond) or via a peptide linker. The two peptides fragments are encoded by amino-acid sequences of different origins. In particular, the fusion protein comprises at least one fragments of bacterial origin and one fragment of virus origin. According to the invention, the fragment of bacterial origin is related to a secretion peptide signal that directs said fusion protein to the T3SS system of a bacterial vector and the fragment of the virus origin is related to a truncated form of the Large T antigen of the Merkel Cell Polyoma virus.

Fusion of the various moieties composing the fusion protein can classically be achieved by a direct peptide bond or by small peptides, advantageously made of at most 1, 2, 3 or 4 amino acids.

The at least two sequences are fused using techniques known to those skilled in the art and, more precisely described in Sambrook et al., 2012, Molecular Cloning: A Laboratory Manual, Fourth Edition, Cold Spring Harbor Laboratory Press.

By "truncated form of the Large T antigen (LT)" it is meant a LT antigen that is shortened compared to the native form of the LT antigen. Therefore, the truncated form of the LT antigen is a fragment of the LT antigen and has a length of less than 817 amino acids.

More specifically, the truncated form of the Large T antigen used in the fusion protein may have an amino acid sequence having at least 80 % identity with one of the amino acid sequences shown in SEQ ID NO:1 which starts with an amino acid in any one of the positions 1 to 5, advantageously in position 2, and ends with an amino acid in any one of the positions 210 to 469, and, preferably, provided that said truncated form of the Large T antigen used in the context of the invention does not bind to the human retinoblastoma protein.

The following expression "the LT truncated form Z has an amino acid sequence shown in SEQ ID NO: Y which starts with the amino acid in any one of positions i to j (i and j are integers and j>i) and ends with the amino acid in any one of positions n to m (n and m are integers and m>n)" means that the LT truncated form Z has any one of the amino acid sequences consisting of the amino acid sequence of SEQ ID NO :Y which starts with any one of the amino acid in positions i to j of the SEQ ID NO :Y and ends with any one of the amino acid in positions n to m of the SEQ ID NO :Y. In particular, the expression encompasses a LT truncated form Z encoded by the SEQ ID NO :Y (i->n) (also called LT(i-n)), a LT truncated form Z encoded by the SEQ ID NO : Y (i->m) (also called LT(i-m)), a LT truncated form Z encoded by the SEQ ID NO :Y (j->n) (also called LT(j-n)) and a LT truncated form Z encoded by the SEQ ID NO :Y (j->m) (also called LT(j-m)).

When it is said that a LT truncated form of the LT antigen of MCPyV has an amino acid sequence, it means that the amino acid sequence of the LT truncated form comprises at least and at most the given defined amino acid sequence.

By "not bind to the human retinoblastoma protein", it is meant that the fusion protein, and more particularly the truncated form of LT, is not able to be associated with the human retinoblastoma protein (pRB) by any attractive interaction, such as non-covalent liaison, in particular hydrogen bond, ionic interactions, Van der Waals forces or hydrophobic bonds; therefore pRB is still able to suppress tumour growth events. The fusion protein is not able to bind to the pRB because the truncated form of the LT protein has either a non-functional human retinoblastoma protein binding site (RBS) or no human retinoblastoma protein binding site at all. The human retinoblastoma protein (abbreviated pRb). is a tumor suppressor protein that is dysfunctional in several major cancers. One function of pRb is to prevent excessive cell growth by inhibiting cell cycle progression until a cell is ready to divide. By "human retinoblastoma protein" it si meant a protein encoded by the RB1 gene (also named RB gene) located on 13q14.1-q14.2 of the human genome and in particular the "human retinoblastoma protein" designates the protein of SEQ ID NO: 26.

In one embodiment, a non-functional human retinoblastoma protein binding site (RBS) may be obtained by introducing mutation in the RBS of the truncated LT antigen when said truncated LT antigen contains at least the first 216 amino acids of the native form of the LT antigen. The RBS located from positions 212 to 216 in SEQ ID NO : 1 is mutated so that it is rendered non-functional, leading to an LT truncated form and a fusion protein unable to bind pRb. While the RBS may be mutated in any one of positions 212 to 216, it is preferred to mutate in position 212, 214 or 216. As a matter of example, a useful mutation is a mutation substituting the glutamic acid in position 216 by another amino acid which is not glutamine, acid aspartic or asparagine. Advantageously, the substitution amino acid is a neutral or positively-charged amino acid. A useful neutral amino acid may be alanine, valine, leucine, isoleucine, methionine, phenylalanine, threonine or tryptophan. A useful positively-charged amino acid may be arginine, histidine or lysine, this latter being preferred. As a matter of example, a useful mutation is a mutation substituting the glutamic acid residue in position 216 by a lysine residue. An example of truncated form of LT having a non-functional RBS is a truncated form of LT having an amino acid sequence shown in SEQ ID NO : 1 which starts with an amino acid in any one of the positions 1 to 5, advantageously in position 2, and ends with the amino acid in any one of the positions 210 to 469 and is further mutated in the human retinoblastoma protein binding site located from positions 212 to 216 in SEQ ID NO : 1. Accordingly, a typical example of an LT truncated form having a non-function RBS (i.e. a LT truncated and mutated form) is LT(2-259, E216→K) by reference to SEQ ID NO : 1. in another embodiment, an LT truncated form having a non-functional RBS may be obtained by substituting any one of the amino acids located from positions 212 to 216 in SEQ ID NO : 1 by a codon stop. Accordingly, a typical example of an LT truncated form having a non-functional RB5 is LT(2-215).

Mutation in SEQ ID NO : 1 may be obtained by molecular techniques well-known in the art and more precisely described in Sambrook et al., 2012, Molecular Cloning: A Laboratory Manual, Fourth Edition, Cold Spring Harbor Laboratory Press.

The ability of the fusion protein to bind or not to the pRB (and the ability to test if a truncated LT form has a non-functional RBS) may be studied by technics well-known by the skilled person in the art such as surface plasmon resonance or immunoprecipitation as described in Shuda et al, Proc. Nat. Acad. Sci (2008), 105 (42):16272.

In a particular embodiment, the truncated form of the LT antigen of the MCPyV of the fusion protein has :
i. an amino acid sequence having at least 80% identity with the amino acid sequence shown on SEQ ID NO: 1 which starts with the amino acid in position 2 and ends with the amino acid in position 215, and, preferably, provided that said truncated form of the LT antigen of the MCPyV does not bind to the human retinoblastoma protein, or
ii. an amino acid sequence having at least 80% identity with the amino acid sequence shown on SEQ ID NO: 1 which starts with the amino acid in position 2 and ends with the amino acid in position 270, and, preferably, provided that said truncated form of the LT antigen of the MCPyV does not bind to the human retinoblastoma protein, or
iii. one of the amino acid sequences shown in SEQ ID NO : 1 which starts with the amino acid in any one of positions 1 to 5, advantageously in position 2, and ends with the amino acid in any one of positions 210 to 215 and, preferably, provided that said truncated form of the LT antigen of the MCPyV does not bind to the human retinoblastoma protein; or
iv. one of the amino acid sequences shown in SEQ ID NO: 1 which starts with the amino acid in any one of positions 1 to 5, advantageously in position 2, and ends with the amino acid in any one of positions 216 to 270, which is further mutated in the human retinoblastoma protein binding site located from positions 212 to 216 in SEQ ID NO : 1, so that said truncated form of the LT antigen of the MCPyV does not bind to the human retinoblastoma protein.

In a particular embodiment, the amino acid sequence of the LT truncated form of the fusion protein expressed, secreted and transferred, preferably translocated by the T3SS bacterial vector according to the invention has at least 80 %, 85 %, 90 %, 95 %, or advantageously 100 % identity, with the amino acid sequence shown in SEQ ID NO : 1 which starts with the amino acid in position 2, and ends with the amino acid in position 469 and, preferably, provided that said LT truncated form is not able to bind the pRB.

In a particular embodiment, the amino acid sequence of the LT truncated form of the fusion protein expressed, secreted and transferred, preferably translocated by the T3SS bacterial vector according to the invention has an amino acid sequence shown in SEQ ID NO : 1 which starts with the amino acid in any one of positions 1 to 5, advantageously in position 2, and ends with the amino acid in position 215.

In a particular embodiment, the amino acid sequence of the LT truncated form of the fusion protein expressed, secreted and transferred, preferably translocated by the T3SS bacterial vector according to the invention has :
∘ one of the amino acid sequences shown in SEQ ID NO : 1 which starts with the amino acid in any one of positions 1 to 5, advantageously in position 2, and ends with the amino acid in any one of positions 250 to 260, advantageously in position 259, which is further mutated in the human retinoblastoma protein binding site located from positions 212 to 216 in SEQ ID NO : 1, so that said LT truncated form does not bind to the human retinoblastoma protein; or
∘ an amino acid sequence having at least 85 % identity with the amino acid sequence shown in SEQ ID NO : 1 which starts with the amino acid in position 2 and ends with the amino acid in position 259 ; and, preferably, provided that LT truncated form does not bind to the human retinoblastoma protein.

In another particular embodiment, the amino acid sequence of the LT truncated form of the fusion protein expressed, secreted and transferred, preferably translocated by the T3SS bacterial vector according to the invention has at least 80 %, 85 %, 90 %, 95 %, or advantageously 100 % identity, with the amino acid sequence shown in SEQ ID NO : 1 which starts with the amino acid in position 2, and ends with the amino acid in position 215 and, preferably, provided that LT truncated form does not bind to the human retinoblastoma protein. A typical example of this is LT(2-215) by reference to SEQ ID NO : 1.

In another particular embodiment, the LT truncated form of the fusion protein expressed, secreted and transferred, preferably translocated by the T3SS bacterial vector according to the invention is defined by an amino acid sequence consisting of an amino acid sequence shown in SEQ ID NO : 1 which starts with the amino acid in any one of positions 1 to 5, advantageously in position 2, and ends with the amino acid in any one of positions 216, 220, 230, 240 or 250 to 270, advantageously in any one of positions 250 to 260, preferably in position 259 ; which is further mutated in the human retinoblastoma protein binding site (RBS) located from position 212 to 216 in SEQ ID NO : 1, so that the fusion protein is unable to bind the human retinoblastoma protein (pRb) and in particular so that said LT truncated form has a non-functional RBS.

For the sake of brevity, the term "LT truncated forms" will herein after includes all the LT truncated forms within the scope of the invention, including the LT truncated and mutated forms and the LT truncated form having no RBS site such as LT truncated forms having an amino acid sequence consisting of the amino acid sequence shown in SEQ ID NO : 1 which starts with the amino acid in any one of positions 1 to 5, advantageously in position 2, and ends with the amino acid in any one of position 210 or 211.

Alternatively, the amino acid sequence of the LT truncated form of the fusion protein expressed, secreted and transferred, preferably translocated, by the T3SS bacterial vector according to the invention has :
- at least 80 %, 85 %, 90 % or 95 % identity, with the amino acid sequence shown in SEQ ID NO : 1 which starts with the amino acid in position 2 and ends with the amino acid in position 270 ; or
- at least 85 %, 90 %, 95 %, 97 % or 99 % identity with the amino acid sequence shown in SEQ ID NO : 1 which starts with the amino acid in position 2 and ends with the amino acid in position 259 ;
and, preferably, provided that said LT truncated form is unable to bind the human retinoblastoma protein (pRb), in particular provided that LT truncated form has a non-functional RBS site.

In yet another embodiment, LT truncated form contained in the fusion protein according to the invention is the sole antigen comprising MCPyV epitopes able to be expressed, secreted and transferred, preferably translocated by the bacterial vector according to the invention.

The fusion protein does not include sequences of LT protein other than the above-defined truncated LT proteins. Therefore, the fusion protein never contains the full-length sequence of the native form of the LT antigen.

Amino acid sequence identity is defined as the percentage of amino acid residues in the variant sequence that are identical with the amino acid residues in the reference sequence after aligning the sequences and if necessary introducing gaps to achieve the maximum percent sequence identity, and not considering any conservative substitution as part of the sequence identity. Sequence identity may be determined in a global manner over the full length of the longest of the sequences being compared which may be the full length of the variant sequence or query sequence, or the full length of the reference sequence.

In practice, global sequence alignment can be achieved and percent identity can be determined by pairwise sequence alignment using algorithms that create an end-to-end optimal alignment (including gaps) of the sequences to be aligned, such as the Needleman-Wunsch algorithm (Needleman et al, J. Mol. Biol. (1970) 48 : 444). The EMBOSS Needle tool uses the Needleman-Wunsch algorithm to read two input sequences (either amino acid or nucleotide sequences) and write their optimal global sequence alignment. This tool and others are accessible on the European Bioinformatics Institute (EBI) web site at http://www.ebi.ac.uk/services and may be used using the default settings.

The LT truncated form useful in the context of the present invention is expressed as a fusion protein which comprises at least one secretion peptide signal able to direct said fusion protein to the type 3 secretion system of the T3SS bacterial vector of the present invention.

The fusion protein comprises an N-terminal moiety which is the secretion peptide signal.

By "secretion peptide signal able to direct the fusion protein to the type 3 secretion system", it is meant a peptide that contains information enabling the fusion protein to be targeted or addressed to the type 3 secretion system and to be secreted from the bacterial vector by the type 3 secretion system of the T3SS bacterial vector. This secretion peptide signal allows the system to distinguish the T3SS-transferred proteins (*i.e.* the fusion protein) from any other proteins of the interior of the bacterial vector (such as the cytosol of the bacterial vector). The function of the secretion peptide signal may be assessed by a secretion test as described in the example.

In one embodiment, the secretion peptide signal is the N-terminal moiety of a T3SS exotoxin (herein after called N-ter Exo).

The T3SS exotoxin may be selected form the group consisting of the N-terminal moiety of a *Pseudomonas* exotoxin *e.g.,* the *Pseudomonas exo5* or *exo*T gene product (ExoS or ExoT), the N-terminal moiety of a *Salmonella* exotoxin, the N-terminal moiety of a *Shigella* exotoxin, the N-terminal moiety of a *Yersinia* exotoxin.

One skilled in the art knows how to choose the secretion peptide signal according to the bacterial vector of the invention (Krall R et al, J Biol Chem. 2004 Jan 23; 279(4):2747-53. Epub 2003 Nov 3.)

In a particular embodiment, the fusion protein comprises an N-terminal moiety which is the N-terminal moiety of a *Pseudomonas* exotoxin e.g., the *Pseudomonas exo5* or *exo*T gene product (herein after called ExoS or ExoT). While such a fusion protein can be expressed in any T3SS bacterial vector, it is convenient to express it in a *Pseudomonas* bacterial vector.

The amino acid sequence of the N-terminal moiety of the *Pseudomonas exo5* gene product (N-ter ExoS) is shown in SEQ ID NO : 2 . It starts with the amino acid in position 1 (Met) of SEQ ID NO : 2 and ends with the amino acids in any one of the positions 15 to 129 of SEQ ID NO : 2, advantageously ends with the amino acid in any one of the positions 15 to 70 of SEQ ID NO : 2, more advantageously ends with the amino acid in any one of the positions 30 to 60 of SEQ ID NO : 2. An example of a useful N-ter ExoS sequence used as secretion peptide signal starts with the amino acid in position 1 (Met) of the SEQ ID NO : 2 and ends with the amino acid in position 54 of the SEQ ID NO : 2. The secretion peptide signal N-ter ExoS (1-54) is herein after referred to as S54.

The amino acid sequence of the N-terminal moiety of the *Pseudomonas exo*T gene product (N-ter ExoT) is shown in SEQ ID NO : 3 . It starts with the amino acid in position 1 (Met) of SEQ ID NO : 3 and ends with the amino acid in any one of the positions 15 to 129 of SEQ ID NO : 3, advantageously ends with the amino acid in any one of the positions 15 to 70 of SEQ ID NO : 3, more advantageously ends with the amino acids in any one of the positions 30 to 60 of SEQ ID NO : 3. An example of a useful N-ter ExoT sequence, as a secretion peptide signal, starts with the amino acid in position 1 (Met) of SEQ ID NO : 3 and ends with the amino acid in position 54 of SEQ ID NO : 3. The secretion peptide signal N-ter ExoT (1-54) is herein after referred to as T54.

In a particular embodiment, the fusion protein can also comprise a moiety able to promote antigen-specific, cell-mediated immunity upon direct antigen delivery to host cells so that the induction of LT-specific CD8 T-cells be triggered. The term "direct antigen delivery," excludes antigen neosynthesis subsequent to nucleotide transfer. A number of proteins or peptides are already known in the art to promote cell-mediated immunity upon direct antigen delivery to host cells. As a matter of example, one may cite the Pan-HLA-DR-binding epitopes (Alexander et al, Immunity (1994) 1 (9) : 751) (hereinafter called PADRE epitope). PADRE epitopes are known to enhance CD8 effectors *via* induction of a CD4+ helper T-cell response which in turn helps inducing a strong CD8+ cytotoxic T-cell response. An example of a PADRE epitope is the peptide having the amino acid sequence as shown in SEQ ID NO : 4. In a preferred embodiment, the PADRE epitope is fused in frame between the secretion peptide signal and any one of the truncated LT forms as defined in the invention.

In another embodiment, the PADRE epitope is fused in frame after any one of the truncated LT forms as defined in the invention. In this case, the PADRE epitope is the C-terminal end of the fusion protein.

In another embodiment, wherein the fusion protein comprising (i) the N-terminal moiety of a T3SS exotoxin (N-ter Exo) *e.g.*, N-ter ExoS or N-ter ExoT, and (ii) any one of the LT truncated forms as defined in the invention, the N-terminal amino acid of the PADRE peptide can be conveniently fused to the C-terminal amino acid of the N-ter Exo (*e.g*., N-ter ExoS or N-ter ExoT, and the C-terminal amino acid of the PADRE peptide can be conveniently fused to the N-terminal amino acid of any one of the LT truncated forms as defined in the invention, thereby leading to the fusion protein called N-ter Exo-PADRE-truncated LT, in particular leading to the fusion proteins called N-ter ExoS-PADRE-truncated LT and N-ter ExoT-PADRE-truncated LT.

In another embodiment, wherein the fusion protein comprising (i) the N-terminal moiety of a T3SS exotoxin (N-ter Exo) *e.g.,* N-ter ExoS or N-ter ExoT, and (ii) any one of the LT truncated forms as defined in the invention, the N-terminal amino acid of the PADRE peptide can be conveniently fused to the C-terminal amino acid of any one of said LT truncated forms as defined in the invention, thereby leading to the fusion protein called N-ter Exo-truncated LT-PADRE, in particular leading to the fusion proteins called N-ter ExoS-truncated LT-PADRE and N-ter ExoT-truncated LT-PADRE.

For clarity's sake, in the context of the bacterial vector technology, it is indicated that there is no need to fuse (to tag) the LT truncated form with a moiety, such as a calreticulin (CRT) tag, able to enhance peptide presentation to MHC class I molecules.

According to a preferred embodiment, the fusion protein comprises, in particular consists (essentially) of (i) a secretion peptide signal as defined in the invention, (ii) a PADRE epitope as defined in the invention, (iii) a LT truncated form as defined in the invention.

According to another preferred embodiment, the fusion protein consists exclusively of (i) a secretion peptide signal as defined in the invention, (ii) a PADRE epitope as defined in the invention, (iii) a LT truncated form as defined in the invention.

All the variants described for the LT truncated form, all variants described for the secretion peptide signal apply to above-preferred embodiments and can be combined together with or without PADRE epitope. Accordingly, useful fusion proteins used in the context of the invention include the N-ter ExoS-truncated LT, N-ter ExoS-PADRE-truncated LT, N-ter ExoT-truncated LT and N-ter-ExoT-PADRE-truncated LT fusion proteins (non-limiting citations).

Another object of the invention concerns an expression cassette, wherein a nucleotide sequence encoding any one of the fusion protein as described in the invention is placed under the control of a promoter.

When the bacterial vector is a bacterium, then it may conveniently comprise an expression cassette, wherein a nucleotide sequence encoding any one of the fusion proteins as described in the invention is placed under the control of a promoter. In a particular embodiment, the promoter can be a promoter inducible by ExsA, such as a promoter of a gene encoding a T3SS exotoxin or a T3SS protein (globally referred to as T3SS promoters). Indeed, in natural conditions, the activity of the genes encoding the T3SS exotoxins (*e.g.*, *exo*S, *exo*T, *exo*U, *exo*Y) and that of the genes encoding the proteins constitutive of T3SS are modulated in trans by the ExsA transcription activator protein. In a particular embodiment, the promoter inducible by ExsA can be the *Pseudomonas exo5* or *exo*T promoter, especially when the secretion peptide signal of the fusion proteins as defined in the invention is the fragment of the N-terminal moiety of the *Pseudomonas exo5* or exoT gene product (N-ter ExoS or N-ter ExoT) respectively. Accordingly, the nucleotide sequence encoding fusion protein as defined in the invention such as the N-ter ExoS-truncated LT or N-ter ExoS-PADRE-truncated LT fusion protein may be placed under the control of the *exo5* promoter. In a similar manner, the nucleotide sequence encoding a fusion protein as defined in the invention such as the N-ter ExoT-truncated LT or N-ter ExoT-PADRE-truncated LT fusion protein is placed under the control of the *exo*T promoter.

In a particular embodiment, when the bacterial vector is a bacterium, then it is able to express both the T3SS complex and the fusion protein as defined in the invention upon a single induction event. To this end, this bacterial vector comprising a first expression cassette, wherein a nucleotide sequence encoding the fusion protein as defined in the invention is placed under the control of an ExsA-inducible promoter, may also comprise a second expression cassette wherein a nucleotide sequence encoding ExsA is placed under the control of an inducible promoter *e.g.* a strongly-inducible promoter. The inducible promoter for ExsA expression may be a reagent-inducible promoter such as a promoter inducible by isopropyl-beta-D-thiogalactopyranoside (IPTG) *i.e.,* the promoter of the lactose operon; in the absence of IPTG, the promoter is repressed. Alternatively, the inducible promoter may be a promoter inducible by a change in culture conditions other than the addition of a reagent, such as a change in temperature (heat-inducible promoter).

When the bacterial vector is a bacterium, it may have been transformed with the expression vector containing the cassette as described hereinabove. It is understood that all transformation techniques known to those skilled in the art can be used such as electroporation, triparental conjugation, bacteriophage transfection, etc. Accordingly, the invention also relates to a process of producing a T3SS bacterial vector being of bacterium, according to the invention and comprising (i) a first expression cassette comprising a sequence encoding a fusion protein as defined in the invention placed under the control of a promoter inducible by ExsA and (ii) a second expression cassette comprising a sequence encoding ExsA placed under the control of an inducible promoter; said process comprising :
(a) Culturing the bacterial vector in a culture medium, under conditions that repress ExsA expression ;
(b) Inducing ExsA expression by a change in culture conditions, advantageously when the culture of the bacterial vector is in exponential phase i.e., mid-exponentional phase;
(c) Optionally adding, a Ca2+ chelator (if not already present in the culture) ; and
(d) Recovering the bacterial vector at the end of the culture.

Addition of a Ca2+ chelator such as ethylene glycol tetraacetic acid (EGTA) or Ethylene diamine tetraacetic acid (EDTA) is advantageous in that it activates the expressed T3SS.

In a particular embodiment of the process according to the invention, the second expression cassette comprises a sequence encoding ExsA placed under the control of an IPTG-inducible promoter; and the process comprises:
(a) Culturing the bacterial vector in the absence of IPTG ;
(b) Inducing ExsA expression by addition of IPTG, advantageously when the culture of the bacterial vector is in exponential phase *i.e.*, mid-exponentional phase;
(c) Optionally adding, a Ca2+ chelator (if not already present in the culture) ; and
(d) Recovering the bacterial vector at the end of the culture.

Advantageously, when the bacterial vector according to the invention is a bacterium, the expression cassette encoding the fusion protein as defined in the invention and/or the expression cassette encoding ExsA can be inserted into the chromosome of said bacterial vector or inserted into a self-replicative vector *e.g.,* a plasmid which may advantageously be a multicopy plasmid.

Advantageously, when the bacterial vector is a bacterium, the maintenance in the bacterial vector of the self-replicative vector may be achieved by inserting into the self-replicative vector an expression cassette for the constitutive expression of an essential protein for the bacterial vector, such as DapD (2,3,4,5-tetrahydropyridine-2,6-dicarboxylate N-succinyltransferase), while the gene encoding this essential protein and naturally present in the bacterial genome has been inactivated *e.g.,* by deletion from the bacterial genome. In this way, only the maintenance of the self-replicative vector in the bacteria guarantees the bacterial survival.

In an advantageous embodiment, when the bacterial vector according to the invention is a bacterium, the bacterial vector according to the invention is unable to substantially replicate in a host organism *e.g.,* a mammal. To this end, the bacterial vector can conveniently be (i) live attenuated or (ii) sensitive to psoralen-induced cross-link upon exposure to long wavelength UVA light, thereby able to give a killed but metabolically active (KBMA) culture of the bacterial vector (upon appropriate photochemical treatment).

Live attenuation can conveniently be achieved by inactivating genes involved in particular metabolic pathways or critical virulence factors, in particular by non-reverting deletions. Aromatic amino acids such as tryptophan (Trp) do not exist in a free form in upper organisms. Accordingly, a bacterial vector auxotroph for such an amino acid cannot efficiently replicate in these organisms. A convenient bacterial vector auxotroph for aromatic amino acids such as Trp, may be a bacterial vector bearing a mutation inactivating the *aro*A gene which encodes the 3-phosphoshikimate 1-carboxyvinyltransferase which is a key enzyme in aromatic synthesis; and thereby lowering the bacterial vector ability for *in vivo* replication and pathogenicity. Such a mutation may be a Δ*aro*A deletion. As a matter of example, it is referred to Δ*aro*A *Pseudomonas* bacteria described in Epaulard et al, Clin. Vacc. Immunol. (2008) 15 (2) : 308, such as CHA-OA (Δ*exoS* Δ*exoT* Δ*aro*A Δ*orf1*) and CHA-OAL (Δ*exoS* Δ*exoT* Δ*aro*A Δ*orf1 Δlasl*). and CLIN-1 (Δ*exoS* Δ*exoT* Δ*oroA* Δ*orf1* Δ*lasl,* adapted for growth in a chemically defined medium as described in WHO 2013/087667). Such auxotroph strain may be available at the Collection Nationale de Culture de Microrganismes (CNCM, Institut Pasteur) under the name CLIN-01 and referenced l-4564 (deposited on December 01, 2011 under the Budapest treaty by the University Joseph Fourier (Grenoble, FR) in relationship with WO 2013/087667. Alternatively, live attenuation can conveniently be achieved by UVA-light induced cross-link in the bacterial chromosome following psoralen treatment of a bacterial vector bearing inactivated *uvr* genes (A and B), which respectively encode the exonucleotidase A and B subunits which are required for nucleotide excision repair. Such a bacterial vector is therefore unable to substantially replicate after a photochemical treatment comprising adding psoralen to a culture of a bacterial vector, culturing the bacterial vector for an additional hour and then submitting the psoralen-treated culture to UVA irradiation. Addition of appropriate doses of psoralen and UVA, to be easily determined by a skilled man, results in a killed but metabolically active (KBMA) culture. By extension of language, the bacterial vector is itself labeled KBMA. As a matter of example, it is referred to the KBMA *Pseudomonas* bacterial vector described in Le Gouëllec et al, Mol. Therapy (2013) 21 (5) : 1076 or WO 2013/087667 (KBMA CHA-OST Δ*exoS* Δ*exoT* Δ*uvrAB*), which also provides for technical details relating to the photochemical treatment.

In yet another particular embodiment, the bacterial vector according to the invention retains sensitivity to antibiotics.

In one embodiment, the T3SS bacterial vector is a bacterium belonging to *Pseudomonas* genus selected from the group consisting in CHA-003 as defined above, CHA-OA as defined above, CHA-OAL as defined above, CHA-OST as defined above and CLIN-01 as defined above. More particularly, the T3SS bacterial vector is the CHA-OST *Pseudomonas* bacterium.

Another object of the present invention concerns a fusion protein which comprises from its N-terminal end to its C-terminal end and fused in frame:
- at least one secretion peptide signal able to direct said fusion protein to the type 3 secretion system of a bacterial vector, when said fusion protein is in a bacterial vector owning a type 3 secretion system, and
- one truncated form of the Large T (LT) antigen of the Merkel Cell Polyomavirus (MCPyV) which has an amino acid sequence having at least 80 % identity with one of the amino acid sequences shown in SEQ ID NO : 1 which starts with an amino acid in any one of positions 1 to 5 and ends with the amino acid in any one of the positions 210 to 469,
and, preferably, provided that said fusion protein does not bind to the human retinoblastoma protein.

All features of the fusion protein able to be expressed, secreted and transferred by the T3SS bacterial vector as defined in the invention described above in connection with the T3SS bacterial vector, apply also for the invention related to the fusion protein.

By "fusion protein is in a bacterial vector", it is meant that the fusion protein is inside of the bacterial vector. The fusion protein may be expressed by the bacterial vector.

In a particular embodiment, the truncated form of the LT antigen of the MCPyV of the fusion protein has :
i. an amino acid sequence having at least 80% identity with the amino acid sequence shown on SEQ ID NO: 1 which starts with the amino acid in position 2 and ends with the amino acid in position 215, and, preferably, provided that said truncated form of the LT antigen of the MCPyV does not bind to the human retinoblastoma protein, or
ii. an amino acid sequence having at least 80% identity with the amino acid sequence shown on SEQ ID NO: 1 which starts with the amino acid in position 2 and ends with the amino acid in position 270, and, preferably, provided that said truncated form of the LT antigen of the MCPyV does not bind to the human retinoblastoma protein, or
iii. one of the amino acid sequences shown in SEQ ID NO : 1 which starts with the amino acid in any one of positions 1 to 5, advantageously in position 2, and ends with the amino acid in any one of positions 210 to 215 and, preferably, provided that said truncated form of the LT antigen of the MCPyV does not bind to the retinoblastoma protein; or
iv. one of the amino acid sequences shown in SEQ ID NO : 1 which starts with the amino acid in any one of positions 1 to 5, advantageously in position 2, and ends with the amino acid in any one of positions 216 to 270, which is further mutated in the human retinoblastoma protein binding site located from positions 212 to 216 in SEQ ID NO : 1, so that said truncated form of the LT antigen of the MCPyV does not bind to the human retinoblastoma protein.

In a particular embodiment, the amino acid sequence of the LT truncated form of the fusion protein according to the invention has an amino acid sequence shown in SEQ ID NO : 1 which starts with the amino acid in any one of positions 1 to 5 , advantageously in position 2, and ends with the amino acid in position 215 and, preferably, provided that said LT truncated form does not bind to the retinoblastoma protein.

In a particular embodiment, the amino acid sequence of the LT truncated form of the fusion protein according to the invention has :
∘ one of the amino acid sequences shown in SEQ ID NO : 1 which starts with the amino acid in any one of positions 1 to 5, advantageously in position 2, and ends with the amino acid in any one of positions 250 to 260, advantageously in position 259, which is further mutated in the human retinoblastoma protein binding site located from positions 212 to 216 in SEQ ID NO : 1, so that said LT truncated form does not bind to the human retinoblastoma protein; or
∘ an amino acid sequence having at least 85 % identity with the amino acid sequence shown in SEQ ID NO : 1 which starts with the amino acid in position 2 and ends with the amino acid in position 259 and, preferably, provided that said LT truncated form does not bind to the human retinoblastoma protein.

In a particular embodiment, the amino acid sequence of the LT truncated form of the fusion protein according to the invention has at least 80 %, 85 %, 90 %, 95 %, or advantageously 100 % identity, with the amino acid sequence shown in SEQ ID NO : 1 which starts with the amino acid in position 2, and ends with the amino acid in position 469 and, preferably, provided that said LT truncated form does not bind to the human retinoblastoma protein is not able to bind the pRB.

In another particular embodiment, the amino acid sequence of the LT truncated form of the fusion protein according to the invention has at least 80 %, 85 %, 90 %, 95 %, or advantageously 100 % identity, with the amino acid sequence shown in SEQ ID NO : 1 which starts with the amino acid in position 2, and ends with the amino acid in position 215 and, preferably, provided that said LT truncated form does not bind to the human retinoblastoma protein. A typical example of this is LT(2-215) by reference to SEQ ID NO : 1.

In another particular embodiment, the LT truncated form of the fusion protein according to the invention is defined by an amino acid sequence consisting of the amino acid sequence shown in SEQ ID NO : 1 which starts with the amino acid in any one of positions 1 to 5, advantageously in position 2, and ends with the amino acid in any one of positions 216, 220, 230, 240 or 250 to 270, advantageously in any one of positions 250 to 260, preferably in position 259 ; which is further mutated in the human retinoblastoma protein binding site (RB5) located from positions 212 to 216 in SEQ ID NO : 1, so that said LT truncated form is unable to bind the human retinoblastoma protein (pRb) and in particular so that said LT truncated form has a non-functional RBS site.

Alternatively, the amino acid sequence of the LT truncated form of the fusion protein according to the invention has:
- at least 80 %, 85 %, 90 % or 95 % identity, with the amino acid sequence shown in SEQ ID NO : 1 which starts with the amino acid in position 2 and ends with the amino acid in position 270 ; or
- at least 85 %, 90 %, 95 %, 97 % or 99 % identity with the amino acid sequence shown in SEQ ID NO : 1 which starts with the amino acid in position 2 and ends with the amino acid in position 259 ;
and, preferably, providing that said LT truncated form does not bind to the human retinoblastoma protein is unable to bind the human retinoblastoma protein (pRb), in particular that said LT truncated form has a non-functional RBS site.

In one embodiment, a non-functional RBS may be obtained by introducing mutation in RBS of the truncated LT antigen when said truncated LT antigen contains at least the first 216 amino acids of the native form of the LT antigen. The RBS located from positions 212 to 216 in SEQ ID NO : 1 is mutated so that it is rendered non-functional, leading to an LT truncated form and a fusion protein unable to bind pRb. While the RBS may be mutated in any of positions 212 to 216, it is preferred to mutate in position 212, 214 or 216 of SEQ ID NO : 1. As a matter of example, a useful mutation is a mutation substituting the glutamic acid in position 216 by another amino acid which is not glutamine, acid aspartic or asparagine. Advantageously, the substitution amino acid is a neutral or positively-charged amino acid. A useful neutral amino acid may be alanine, valine, leucine, isoleucine, methionine, phenylalanine, threonine or tryptophan. A useful positively-charged amino acid may be arginine, histidine or lysine, this latter being preferred. As a matter of example, a useful mutation is a mutation substituting the glutamic acid residue in position 216 by lysine residue. An example of truncated form of LT having a non-functional RBS is a truncated form of LT having an amino acid sequence shown in SEQ ID NO : 1 which starts with an amino acid in any one of the positions 1 to 5, advantageously in position 2, and ends with the amino acid in any one of the positions 210 to 469 and is further mutated in the RBS located from position 212 to 216 in SEQ ID NO: 1. Accordingly, a typical example of an LT truncated form having a non-function RBS site (i.e a LT truncated and mutated form) is LT(2-259, E216→K) by reference to SEQ ID NO : 1. In another embodiment, an LT truncated form having a non-functional RBS may be obtained by substituting any one of the amino acids located from position 212 to the 216 in SEQ ID NO : 1 by a codon stop. Accordingly, a typical example of an LT truncated form having a non-functional RBS is LT(2-215).

For the sake of brevity, the term "LT truncated forms" will herein after include all the LT truncated forms within the scope of the invention, including the LT truncated and mutated forms and the LT truncated form having no RBS site such as LT truncated forms having an amino acid sequence consisting of the amino acid sequence shown in SEQ ID NO : 1 which starts with the amino acid in any one of positions 1 to 5, advantageously in position 2, and ends with the amino acid in any one of positions 210 or 211.

In a preferred embodiment, the fusion protein comprises LT truncated form, which is the sole antigen comprising MCPyV epitopes able to be expressed, secreted and transferred, preferably translocated by the bacterial vector according to the invention.

The fusion protein of the invention does not include sequences of LT protein other than the above-defined truncated LT proteins. Therefore, the fusion protein of the invention never contains the full-length sequence of the native form of the LT antigen.

The fusion protein of the present invention comprises at least one secretion peptide signal able to direct said fusion protein to the type 3 secretion system of a T3SS bacterial vector, as defined in the invention. All features related to the secretion peptide signal that have been described in relation with the bacterial vector apply here.

In a particular embodiment, the fusion protein can also comprise a moiety able to promote antigen-specific, cell-mediated immunity upon direct antigen delivery to host cells so that the induction of LT-specific CD8 T-cells may be triggered. All features related to said moiety that have been described in relation with the bacterial vector apply here.

For clarity's sake, it is indicated that there is no need to fuse (to tag) the LT truncated form with a moiety, such as a calreticulin (CRT) tag, able to enhance peptide presentation to MHC class I molecules.

According to a preferred embodiment, the fusion protein comprises, in particular consists (essentially) of (i) a secretion peptide signal as defined in the invention, (ii) a PADRE epitope as defined in the invention, (iii) a LT truncated form as defined in the invention. According to another preferred embodiment, the fusion protein consists exclusively of (i) a secretion peptide signal as defined in the invention, (ii) a PADRE epitope as defined in the invention, (iii) a LT truncated form as defined in the invention.

All the variants described for the LT truncated form, and all the variants described for the secretion peptide signal apply to above-preferred embodiments and can be combined together with or without PADRE epitope.

In a preferred embodiment, the fusion protein is the S54-PADRE-LT(2-259, E216→K) protein or S54-PADRE-LT(2-215) protein, wherein the S54 moiety, PADRE epitope, LT(2-259,E216→K) and LT(2-215) are as described in the invention.

Yet another aspect of the invention relates to an isolated polynucleotide encoding the fusion protein of the invention. The polynucleotide is a synthetic or recombinant DNA either single- or double-stranded.

Preferably, the isolated polynucleotide comprises a coding sequence which is optimized for the T3SS bacterial vector, selected for the secretion, transfer, preferably translocation, of the fusion protein, owning a type 3 secretion system in which the fusion protein of the invention is expressed. All features related to the T3SS bacterial vector as defined in the invention, apply here. In a preferred embodiment, the T3SS vector is a bacterium belonging to the *Pseudomonas* strain. As an example, a nucleic acid codon-optimized if at least one codon in the isolated polynucleotide is replaced with codon that is more frequently used by *Pseudomonas* for that amino acid than the codon in the original sequence. Said isolated polynucleotide may be introduced in an expression cassette in order to be expressed in a T3SS bacterial vector as defined in the invention.

The T3SS bacterial vector as defined in the invention is genetically modified for inducing an expression cassette for the expression of the fusion protein.

In a preferred embodiment of the bacterial vector as defined in the invention, said polynucleotide is inserted under the control of promoter into an expression cassette such as a plasmid. The plasmid is capable of expressing said polynucleotide when transfected or transformed into a bacterial vector owning a type 3 secretion system. Plasmids are known in the art, and a number of them are commercially available, but it is also possible to construct or modify them using genetic manipulation techniques.

In a particular embodiment, the promoter can be a promoter inducible by ExsA, such as a promoter of a gene encoding a T3SS exotoxin or a T3SS protein (globally referred to as T3SS promoters). In a particular embodiment, the promoter inducible by ExsA can be the *Pseudomonas exo5* or *exo*T promoter, especially when the fusion protein of the invention comprises an N-terminal moiety which is the fragment of the N-terminal moiety of the *Pseudomonas exo5* or *exo*T gene product (N-ter ExoS or N-ter ExoT) respectively. Accordingly, the nucleotide sequence encoding a fusion protein such as the N-ter ExoS-truncated LT or N-ter ExoS-PADRE-truncated LT fusion product may be placed under the control of the *exo*S promoter. In a similar manner, the nucleotide sequence encoding a fusion protein such as the N-ter ExoT-truncated LT or N-ter ExoT-PADRE-truncated LT fusion product, is placed under the control of the *exo*T promoter.

The bacterial vector as described in the invention may be transformed with the plasmid described hereinabove.

It is understood that all transformation techniques known to those skilled in the art can be used such as electroporation, triparental conjugation, bacteriophage transfection, etc.

Under another aspect, the invention relates to:
(I) A pharmaceutical composition comprising a bacterial vector according to the invention
(II) A bacterial vector according to the invention for use as a medicament;
(III) A bacterial vector according to the invention for use as a medicament in the treatment of cancer;
(IV) A bacterial vector according to the invention for use in a method of combating or preventing MCPyV infection ;
(V) The use of a bacterial vector according to the invention, for the manufacture of a medicament for combating or preventing MCPyV infection; and
(VI) A method of combating or preventing MCPyV infection which comprises administering to a patient in need, a bacterial vector according to the invention or a pharmaceutical composition comprising a bacterial vector according to the invention.

MCPyV infection can be combated or prevented by promoting in a patient a CD8+ immune response against cells infected with MCPyV, including (i) cells expressing whole or part of the MCPyV LT antigen, and (ii) cells (a) expressing whole or part of the MCPyV LT antigen and (b) in the genome of which is integrated an MCPyV nucleotide sequence encoding said whole or part of the LT antigen. The immune response against cells infected with MCPyV is advantageously LT-specific.

By "CD8+ immune response" is meant an immune response characterized by the induction of CD8 T-cells, in particular CD8 cytotoxic T-cells. CD8 T-cells express the CD8 glycoprotein at their membrane surface. The induction of CD8 cytotoxic T-cells i.e., LT-specific CD8 cytotoxic T-cells, is effective in significantly reducing the amount of cells expressing whole or part of the LT antigen.

MCPyV infection may be asymptomatic or not. Accordingly, cells infected with MCPyV may be in a tumoral state or not. In asymptomatic patients, non-tumoral cells infected with MCPyV, in particular those expressing whole or part of the LT antigen, are nevertheless indicative of a pre-tumoral status and it is therefore desirable to eliminate them because the patients are indeed at risk of developing a tumor. In addition to this, cells (a) expressing whole or part of the MCPyV LT antigen and (b) in the genome of which is integrated an MCPyV nucleotide sequence encoding said whole or part of the LT antigen are indicative of a higher risk of developing a tumor.

Accordingly, the bacterial vector according to the invention or the pharmaceutical composition comprising a bacterial vector according to the invention, can also be for use in :
(i) A method of preventing tumor appearance in asymptomatic patients infected with MCPyV.
(ii) A method of reducing the growth or propagation of a tumor in a patient suffering from a tumoral disorder characterized by the presence of tumoral cells expressing the LT antigen of MCPyV.
(iii) A method of treating a tumoral disorder characterized by the presence of tumoral cells expressing the LT antigen of MCPyV.

Accordingly, the invention also relates to any one of methods (i) to (iii) specified above, wherein the bacterial vector according to the invention is administered to a patient in need.

Accordingly, the invention also relates to any one of methods (i) to (iii) specified above, wherein the pharmaceutical composition comprising the bacterial vector according to the invention is administered to a patient in need.

MCPyV infection, asymptomatic or not, may be revealed by PCR amplification of viral DNA from a blood sampling. PCR amplification may be conventionally achieved by a man skilled in the art. Amplification of MCPyV DNA may be achieved using PCR sense and antisense primers respectively shown in SEQ ID NO : 11 and 12.

Alternatively, when possible, cell sampling may be achieved in asymptomatic patients associated with a particular risk factor *e.g.*, a broncho-alveolar lavage in asymptomatic smokers or biopsy in asymptomatic patients with family antecedents for a particular type of cancer. This cell sampling (*e.g.,* biopsy) followed by the search for LT expression and viral DNA integration into the cell genome, will indicate the risk level of developing a tumor.

LT expression may be revealed *i.e.*, by (i) detecting the presence of the LT antigen in the cell extract by Western blot or (ii) by immunohistochemistry. Those techniques are known in the art and may conventionally be achieved by a man skilled in the art. Those techniques require the use of a monoclonal antibody generated against MCPyV LT antigen residues 1-260 such as monoclonal antibody CM2B4 supplied by Santa Cruz Biotech Inc. under reference sc-136172 or monoclonal antibody Ab3 described in Rodig et al, J. Clin. Invest. (2012) 122: 4645.

Viral DNA integration may be revealed by isolating the cell DNA and detecting the viral DNA by PCR amplification using PCR sense and antisense primers respectively shown in SEQ ID NO : 11 and 12.

In cancer patients, the MCPyV infection may also be detected upon cell sampling *e.g.,* biopsy or blood sampling followed by the showing of LT expression and viral DNA integration into the cell genome, using the methods reported herein above.

Tumoral disorders intended for prevention or treatment, may be *i.e.,* any one of Merkel cell carcinoma, neuro-endocrine cutaneous carcinoma, squamous cell carcinoma, basal cell carcinoma, Bowen's disease, cutaneous B-cell lymphoma, cutaneous T-cell lymphoma, chronic lymphocytic leukemia, small lymphocytic lymphoma, human central nervous system tumors, chronic lymphotic leukemia, glioblastoma, cervical carcinoma, large cell lung carcinoma, lung adenocarcinoma or small cell lung cancer.

A pharmaceutical composition according to the invention *i.e.*, be a vaccinal composition, comprises a bacterial vector according to the invention together with a pharmaceutically acceptable diluent or carrier. Accordingly, the bacterial vector can be formulated for parenteral administration *e.g.,* in a pharmaceutically acceptable diluent. Alternatively, the bacterial vector can be lyophilized for storage and extemporaneously diluted in a pharmaceutically acceptable diluent, prior use.

The method of combating or preventing MCPyV infection or any one of the methods (i) to (iii) according to the invention can comprise parenterally, *i.e.,* subcutaneously, intradermally or intravenously, administering the bacterial vector of the invention or a pharmaceutical composition of the invention to a patient in need. Conveniently, a dose of a bacterial vector of the invention may be administered one or several times a day, week or month interval. As illustrative example only, it is indicated that a dose may comprise from 1 x 10⁶ to 1 x 10¹⁰ colony-forming units (cfu) when a bacterial vector is administrated.

As a matter of non-limiting example, a bacterial vector according to the invention may be a *Pseudomonas* bacterial vector unable to express ExoS, ExoT and ExoU and able to express, secrete and transfer, preferably translocate *via* the T3SS complex, the fusion protein S54-PADRE-LT(2-259, E216→K) or S54-PADRE-LT(2-215), wherein the S54 moiety, PADRE epitope, LT(2-259,E216→K) and LT(2-215) are as described herein above. This is further illustrated in the Experimental Section below.

### Figures

Figure 1 is a schematic representation of plasmid pEAI-S54 described in Wang et al, 2012.
Figure 2 shows a Western blot analysis of the supernatant of an LT^{VAX} culture, using an LT-specific monoclonal antibody.
Figure 3 shows the IFNg release (pg/ml) by NP68-specific CD8 T-cells co-cultivated with DCs previously incubated with LT^{VAX}, as measured by an ELISA assay.
Figure 4 shows the IFNg release (pg/ml) by SIINFEKL-specific CD8 T-cells co-cultivated with B16 LT tumor cells, as measured by an ELISA assay.
Figure 5 shows the tumor growth in B16 LT tumor cell-bearing mice treated with LT^{VAX}

### Experimentals

### Materials and Methods

### DNA cloning

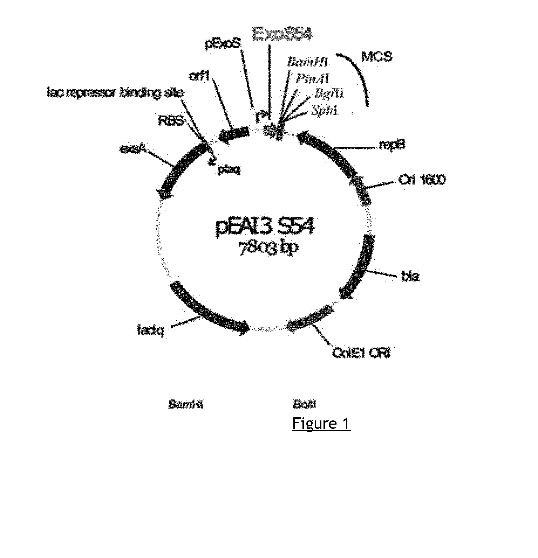

DNA sequence that encodes the LT protein of Merkel Cell Polyomavirus (MCRyV) as shown in SEQ ID NO : 1 was optimized *in silico* for expression in *Pseudomonas aeruginosa.* The DNA sequence that encodes for the LT truncated form LT(2-215) is shown in SEQ ID NO : 22. The DNA sequence that encodes for the LT truncated and mutatetd form LT(2-259,E216->K) is shown in SEQ ID NO : 7.. These sequences were cloned in the monocistronic pEAI-S54-PADRE plasmid (Wang et al, 2012 ; Figure 1) downstream the nucleotide PADRE sequence (SEQ ID NO : 9). This generated the plasmids pEAI-S54-PADRE-LT(2-215) and pEAI-S54-PADRE-LT(2-259, E216->K) able to express the fusion protein S54-PADRE-LT(2-215) and S54-PADRE-LT(2-259, E216->K), respectively.

DNA sequence (SEQ ID NO : 7) that encodes the amino acid sequence (2-259, E216->K) was also cloned in the same monocistronic pEAI-S54-PADRE plasmid downstream the nucleotide PADRE sequence (SEQ ID NO : 9) together with the sequence encoding the NP68 peptide (as shown in SEQ ID NO : 10). This generated the plasmid pEAI-S54-PADRE-LT(2-259, E216->K)-NP68 able to express the fusion protein S54-PADRE-LT(2-259,E216->K)-NP68. The NP68 peptide (SEQ ID N°5) which corresponds to amino acids 362-378 of the nucleoprotein (NP) protein of the influenza virus strain A/Nt/60/68, was used as a tag for monitoring the immune response against the expression product.

Cloned sequence was verified by DNA sequencing.

Plasmids pEAI-554-PADRE-LT(2-215) and pEAI-S54-PADRE-LT(2-259, E216-K) have been used for the secretion assay. These plasmids encode for the fusion protein S54-PADRE-LT(2-215) and S54-PADRE-LT(2-259, E216->K) respectively.

The plasmid pEAI-S54-PADRE-LT(2-259, E216->K)-NP68 has been used for the generation of LT^{VAX} as this plasmid permitted the in vitro validation of antigen delivery thanks to the presence of NP68. This plasmid encodes for the fusion protein S54-PADRE-LT(2-259,E216->K)-NP68.

For the generation of the B16 LT tumor cells, the LT-IRES-GFP plasmid was constructed to express under the control of the EF1a promoter, (i) the LT(1-259, E216->K) tagged in C-ter by the ovalbumine peptide (257-264) SIINFEKL (SEQ ID NO : 6) and (ii) the Green Fluorescent Protein (GFP). In the pCDNA3.1 plasmid (Life Technologies) devoid of the CMV promoter, the following elements were inserted from 5' to 3' : the EF1a promoter (SEQ ID NO: 25), the DNA sequence encoding LT(1-259, E216->K) (SEQ ID NO : 23), the DNA sequence encoding the SIINFEKL peptide, an Internal Ribosomal Entry Site (IRES; SEQ ID NO : 24) and the DNA sequence encoding GFP (SEQ ID NO: 21) in order to obtain the LT-IRES-GFP plasmid. The SIINFEKL peptide was used as a tag for monitoring the immune response against the B16 LT cell line.

### Generation of LT^{VAX}

The LT^{VAX} bacterial vector was generated by transforming the *P. aeruginosa,* attenuated strain CHA-OST (Epaulard et al, 2006) with the plasmids pEAI-554-PADRE-LT(2-215), pEAI-S54-PADRE-LT(2-259, E-216->K) or the plasmid pEAI-S54-PADRE-LT(2-259, E216->K)-NP68. Briefly, the CHA-OST strain was incubated in Luria Bertani broth (Miller) at 37°C, 225 rpm agitation for 16 hours. 2 ml of bacterial culture were resuspended in 300 mM sucrose solution (Euromedex). After 2 washes, 1/10 of the bacterial population was incubated for 20 minutes at 4°C with 100 ng of plasmid DNA. Electroporation was performed at 1.8 kV for 5 milli seconds. After a 1 hour incubation, at 37°C, 225 rpm in SOC (Super Optimal Catabolite) medium (Life Technologies), bacteria were plated on *Pseudomonas* isolation agar (PiA) medium (Gibco) supplemented with 300 µg/ml carbenicillin (Sigma).

### Culture of LT^{VAX}

LT^{VAX} was incubated at 37°C with 300 rpm agitation, in a chemically defined medium based on the glucose minimal medium (M9) supplemented with magnesium and calcium, named MM9 medium (Le Gouëllec et al, 2013). Precisely, MM9 contains : extract of synthetic yeast without tryptophan 4 g/L (Sigma); FeSO₄ 0.4 g/L (Sigma); glucose 2.5 g/L (Euromedex) ; glycerol 1% (Euromedex) ; Citric acid 0.36 g/L (Euromedex); M9 Salts Medium 5X (Sigma).

The expression of the fusion protein S54-PADRE-LT(2-215), S54-PADRE-LT(2-259, E216-K) S54-PADRE-LT(2-259, E216->K)-NP68 and T3SS was induced by addition of isopropyl-beta-D-thiogalactopyranoside (IPTG) and the T3SS function was activated by Ca2+ chelation with ethylene glycol tetraacetic acid (EGTA). Briefly, LT^{VAX} was then diluted at Optical Density (OD₆₀₀) of 0.2 in MM9 medium supplemented with 300 µg/mL carbenicillin, 1.6 mM IPTG, 5 mM EGTA and 20 mM MgCl₂ (all from Euromedex), and incubated at 37°C with 300 rpm agitation until OD₆₀₀ 1.6 was reached. LT^{VAX} was then resuspended in MM9 medium, ready for in vivo or in vitro use.

### Protein Secretion Assay

The LT^{VAX} culture was centrifuged at 13 000 g for 10 minutes. In order to precipitate proteins, the supernatant was incubated with 20% (v/v) trichloroacetic acid at 4°C for 10 minutes, then centrifuged at 15000 g, 4°C for 5 minutes and washed twice with acetone (Sigma), centrifuging at 15000 g for 5 minutes after each wash. Proteins were resuspended in 80 µl of denaturation buffer (0.5 M Tris-HCL, 0.6 M DTT, 10% SDS, 0.012% bromophenol blue, 15% glycerol) and migrated in SDS-PAGE in a 10% polyacrylamide gel (Ready Gels Precast Gel, Biorad) following manufacturer's instructions.

### Western blot analysis

Proteins were loaded in 12% acrylamide gel (Promega), and run in running buffer (Promega). Protein transfer was performed at 120 V for 60 minutes with the precast system (Promega). Anti-LT antibodies (2000-fold dilution, purchased from Santa Cruz Biotech ; ref : sc-136172) and anti-mouse horse radish peroxidase secondary antibody (10⁴ dilution, from Dutscher) were used.

### Cell lines and primary cells

B16F0 cells (ATCC-CRL-6322) and B16LT tumor cells were cultured in DMEM medium, supplemented with 10% FBS, penicillin (100 U/ml), streptomycin (100 U/ml), 1 mM glutamine, and 1 mM sodium pyruvate (all items from Life Technologies).

Primary dendritic cells (DCs) were obtained as follows : bone marrow from tibia and femurs of C57BL/6 mice was flushed and cultured in RPMI 1640 medium supplemented with 10% heat-inactivated foetal bovine serum (FBS), 2 mM L-glutamine, 10 mM HEPES buffer, 50 µg/ml gentamicin (all from Life Technologies) and 50 µM 2-Mercaptoethanol (Sigma-Aldrich). Red blood cells were lysed by resuspending pelleted cells in Tris-ammonium chloride (Life Technologies) for 2 minutes. Cells were then resuspended in culture medium and cultured for 9 days at 10⁶ cells/ml in six-well plate, at 37°C and 7% CO₂, in culture medium supplemented with 200 ng/ml recombinant human Flt3 ligand (Amgen) for monocyte differentiation into DCs.

Activated, anti-NP68 T-cells were obtained from splenocytes of F5 transgenic mice (Mamalaki et al, 1993). Spleen was collected aseptically and splenocytes were cultured in DMEM supplemented with 6% FBS, 2 mM L-glutamine, 10 mM HEPES buffer, 50 µM 2-Mercaptoethanol, 10% recombinant IL-2 and 10 nM NP68 peptide (ProteoGenix). After 5 days of culture, cells were harvested and cultured for two additional days in culture medium, in the absence of peptide NP68.

Activated, anti-ovalbumine peptide SIINFEKL T-cells were obtained from splenocytes of OT1 transgenic mice (Hogquist et al, 1994 ; Clarke et ai, 2000). Spleen was collected aseptically and splenocytes were cultured in DMEM supplemented with 6% FBS, 2 mM L-glutamine, 10 mM HEPES buffer, 50 µM 2-Mercaptoethanol and 10 nM SIINFEKL peptide (ProteoGenix). After 5-day culture, cells were harvested and cultured for two additional days in culture medium, in the absence of peptide SIINFEKL.

### in vitro validation of LT^{VAX} antigen delivery

Primary dendritic cells (DCs) were seeded in 96-well plate at 10⁵ cells/well in 100 µl of medium. DCs were co-incubated with LT^{VAX} or BacVac^{™} vector (*Pseudomonas* strain CHA-OST transformed with the pEAI-554-PADRE plasmid) at multiplicity of infection of 0.1. After 1 hour, bacteria were removed by performing two washes with DC culture medium, and incubating DCs twice (30 minutes at each time) in medium enriched with gentamycin (Life Technologies). Activated, anti-NP68 T-cells were co-incubated with DCs at ratio 1:1 and incubated at 37°C, 7% CO₂. Co-culture supernatants were collected 16 hours later. IFNg levels measured in the co-culture supernatants were quantified by ELISA (ReD systems) following manufacturer's instructions.

### Generation of the B16 LT tumor cell line

B16F0 tumor cells were transfected with the LT-IRES-GFP plasmid, using the jetPEI^{™} transfection reagent (PolyPlus) following manufacturer's instructions to give B16F0 tumor cells expressing LT(1-259, E216->K) (now called B16 LT tumor cells). Stable plasmid integration in the eukaryotic cell genome was obtained by incubating B16 LT tumor cells in medium supplemented with 10% FBS and 0.1 g/L G418 (Sigma). Surviving, GFP-positive tumor cell clones were isolated.

### Validation of the B16 LT GFP-positive cells for use in the tumor model

B16 LT, GFP-positive tumor cells (and B16F0 tumor cells) were respectively co-seeded with activated, SIINFEKL-specific T-cells, at ratio 1:1 and co-incubated at 37°C, 7% CO₂. Co-culture supernatants were collected 16 hours later. IFNg levels were quantified by ELISA (ReD systems) following manufacturer's instructions.

### Tumor challenge experiment

Female C57BL/6j mice were purchased from Janvier SA (Le Genest-Saint-Isle, France) and kept under pathogen-free conditions in the animal facility of the University Joseph Fourier (Grenoble, France). Experiments were approved by the Animal Experiment Committee of the Region and were performed in accordance with institutional and national guidelines. 2x10⁵ B16 LT tumor cells were resuspended in PBS and administered subcutaneously into the flank of 6-8 week old mice. Mice were monitored for tumor appearance every 24 hours. LT^{VAX} injection was performed subcutaneously (5*10⁵ bacteria for the first two injections, then 10⁶ for the following injections) when tumor became palpable (5-7 days post inoculation) and then every three-four days throughout the experiment. Groups of 6 mice per condition were used. Tumor size was measured by using a caliper. Two measures were performed.

### Results

### LT^{VAX} efficiently secretes fusion proteins comprising the truncated form of LT (2-215) or the truncated form of LT(2-259, E216→K)

LT^{VAX} is an immunotherapy product based on the BacVac^{™} technology (Epaulard 2006). LT^{VAX} was obtained by transforming a BacVac^{™} vector (CHA-OST strain of *P.aeruginosa)* with a plasmid encoding a fusion product of (i) the *P.aeruginosa* ExoS peptide (1-54), also called the S54 peptide, and (ii) a mutated, truncated form of the LT antigen of MCPγV, hereinafter called LT (2-259, E216→K). The E216 →K mutation was introduced to inactivate RBS so that the LT product (in particular the fusion protein) encoded by the plasmid is substantially non-oncogenic. The encoding sequence was optimized for expression in *P. aeruginosa.* Alternatively, LT^{VAX} was obtained by transforming the BacVac^{™} vector with a plasmid encoding a fusion product of (i) the S54 peptide, and (ii) a mutated, truncated form of the LT antigen of MCPγV, hereinafter called LT (2-215) This truncation generates an RBS so that the LT product (in particular a fusion protein) encoded by the plasmid is substantially non-oncogenic. The encoding sequence was optimized for expression in *P. aeruginosa.*

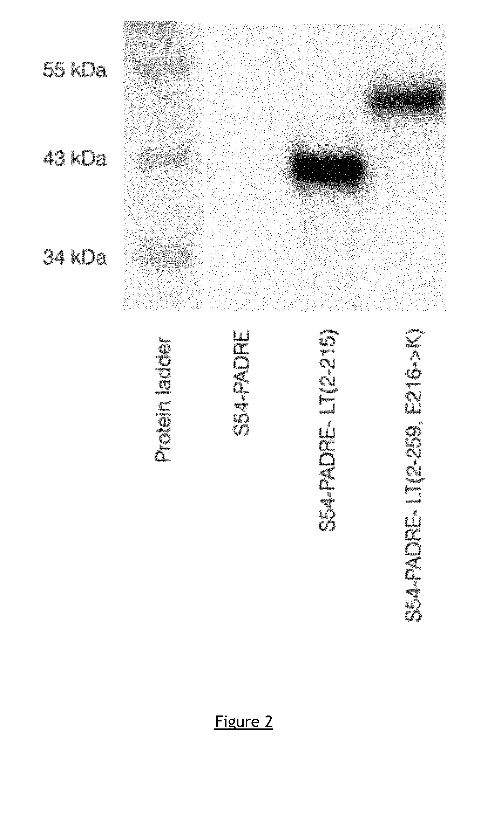

LT^{VAX} were cultured and incubated with (i) IPTG to express the *exs*A gene product which in turn activates the *exo*S promoter; and (ii) EGTA, to trigger delivery of the fusion protein S54-PADRE-LT(2-259, E216->K) or S54-PADRE-LT(2-215) *via* the T3SS. As shown in Figure 2, Western blot analysis of the supernatant of an LT^{VAX} culture, using an LT-specific monoclonal antibody, demonstrates that the fusion protein comprising the LT (2-259, E216→K) and the LT(2-215) were efficiently expressed and secreted *via* the T3SS, thanks to the 554 peptide. Secretion of the fusion protein comprising the LT (2-259, E216→K) and the LT(2-215) were not observed in the control test (BacVac^{™} transformed with the pEAI-554-PADRE plasmid).

Moreover, secretion tests as described above have also been performed with the following fusion proteins:
- S54-LT(2-817) which corresponds to a fusion protein comprising the 554 peptide and the native form of the LT antigen of the MCPγV having the amino acid sequence shown in SEQ ID NO : 13 which starts to the amino acid in position 2 and ends with the amino acid in position 817. The nucleic sequence shown in SEQ ID NO : 14 which has been optimized for expression in *P. aeruginosa* has been used for the construction of said fusion protein. The nucleic sequence shown in SEQ ID NO : 8 which encodes for the S54 peptide has also been used for the construction of said fusion protein.
- S54-PADRE-sT(2-186) which corresponds to a fusion protein comprising the S54 peptide, the PADRE epitope and the native form of the Small T (sT) antigen of the MCPγV having the amino acid sequence shown in SEQ ID NO : 15 which starts to the amino acid in position 2 and ends with the amino acid in position 186. The nucleic sequence shown in SEQ ID NO : 16 which has been optimized for expression in *P. aeruginosa* has been used for the construction of said fusion protein. The nucleic sequence shown in SEQ ID NO : 8 which encodes for the S54 peptide and the nucleic sequence SEQ ID NO : 9 which encodes for the PADRE epitope have also been used for the construction of said fusion protein.
- S54-PADRE-sT(81-186) which corresponds to a fusion protein comprising the S54 peptide, the PADRE epitope and the sT truncated form having the amino acid sequence shown in SEQ ID NO : 15 which starts to the amino acid in position 81 and ends with the amino acid in position 186. The nucleic sequence shown in SEQ ID NO : 17 which has been optimized for expression in *P*. *aeruginosa* has been used for the construction of said fusion protein. The nucleic sequence shown in SEQ ID NO : 8 which encodes for the S54 peptide and the nucleic sequence SEQ ID NO : 9 which encodes for the PADRE epitope have also been used for the construction of said fusion protein.
- S54-PADRE-sT(2-141) which corresponds to a fusion protein comprising the S54 peptide, the PADRE epitope and the sT truncated form having the amino acid sequence shown in SEQ ID NO : 15 which starts to the amino acid in position 2 and ends with the amino acid in position 141. The nucleic sequence shown in SEQ ID NO : 18 which has been optimized for expression in *P. aeruginosa* has been used for the construction of said fusion protein. The nucleic sequence shown in SEQ ID NO : 8 which encodes for the S54 peptide and the nucleic sequence SEQ ID NO : 9 which encodes for the PADRE epitope have also been used for the construction of said fusion protein.
- S54-PADRE-sT(81-141) which corresponds to a fusion protein comprising the S54 peptide, the PADRE epitope and the sT truncated form having the amino acid sequence shown in SEQ ID NO : 15 which starts to the amino acid in position 81 and ends with the amino acid in position 141. The nucleic sequence shown in SEQ ID NO : 19 which has been optimized for expression in *P*. *aeruginosa* has been used for the construction of said fusion protein. The nucleic sequence shown in SEQ ID NO : 8 which encodes for the S54 peptide and the nucleic sequence SEQ ID NO : 9 which encodes for the PADRE epitope have also been used for the construction of said fusion protein.

The Small T (sT) antigen is an oncoprotein expressed by MCPγV. The first 79^{th} amino acid of sT antigen are identical to the first 79^{th} amino acid of LT antigen.

Said fusion proteins (S54-LT(2-817)), (S54-PADRE-sT(2-186)), (S54-PADRE-sT(81-186)), (S54-PADRE-sT(2-141)), (S54-PADRE-sT(81-141)) are expressed by the bacterial vector LT^{VAX} but none of them secrete and transfer outside of the bacterial vector despite the presence of the secretion peptide signal S54.

These experiments demonstrate that it is not easy to predict whether a fusion protein can be secreted and transferred, preferably translocated, by a bacterial vector owning a type 3 secretion system.

### DCs that have received LT (259, E216->K) thanks to the vector LT^{VAX} are efficient for LT presentation to T-cells

LT^{VAX} delivers the fusion protein comprising the LT truncated form (2-259, E216->K) antigen tagged by the NP68 peptide. This peptide is frequently used in immunology research to sensitively trace the immune response. This is possible by using T-cells derived from F5 transgenic mice, which have high frequency of NP68-specific T-cells. This tool is particularly useful as there is no immunomonitoring tool specific for LT available on the market.

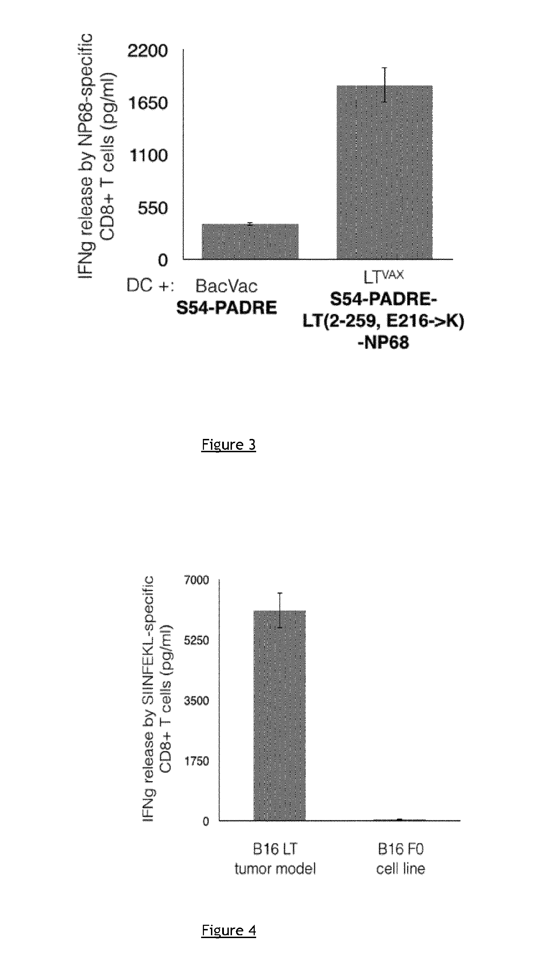

As shown in Figure 3, IFNg release is significantly higher when activated NP68-specific T-cells were co-cultivated with DCs previously co-cultivated with LT^{VAX} compared to the negative control (NP68-specific T-cells co-cultivated with DCs previously co-cultivated with BacVac^{™}). This reveals that the fusion protein comprising the LT truncated form (2-259, E216→K) as delivered by LT^{VAX} to DCs was efficiently processed for presentation to CD8 T-cells.

### B16 LT tumor cells are efficient for LT presentation to T-cells

The LT-IRES-GFP plasmid used for the generation of the B16 LT tumor cells expresses an mRNA encoding two distinct proteins : (i) LT (2-259, E216→K) tagged by the SIINFEKL peptide and (ii) GFP. The plasmid also expresses the gene conferring resistance to geneticin. Following the isolation of cells that stably integrated the plasmid in the genome by prolonged treatment with geneticin, GFP-positive clones were isolated.

The SIINFEKL peptide was tagged to LT (2-259, E216→K) in order to confirm that LT (2-259, E216→K) is expressed and processed by the B16 LT tumor cells for presentation to T-cells. Consistently with this hypothesis, IFNg was released by activated, SIINFEKL-specific CD8 T-cells, when co-incubated with B16 LT tumor cells (Figure 4). Instead, no IFNg was detected when the same T-cells were co-incubated with the parental tumor cell line (B16F0). This demonstrates that the B16 LT tumor cells properly express LT (2-259, E216→K) and processed it for presentation to CD8 T-cells.

### LT^{VAX} is efficient for tumor growth control

The efficacy of LT^{VAX} was demonstrated in an *in vivo* tumor challenge experiment: B16 LT tumor cells were implanted in mice, and LT^{VAX} treatment was performed when the tumor became palpable, and then repeated every three-four days throughout the experiment. LT^{VAX} significantly reduced the tumoral growth (p<0.5, T test), with a reduction of the tumor size of approximately 75 % relative to tumor-bearing mice treated with the BacVac^{™} control vector (delivering no antigen), as measured 19 days post tumor implantation (Figure 5).

### Sequences

**SEQ ID NO : 1,** Truncated LT from position 1 to 469
   3-letter code : 
Met Asp Leu Val Leu Asn Arg Lys Glu Arg Glu Ala Leu Cys
Lys Leu Leu Glu Ile Ala Pro Asn Cys Tyr Gly Asn Ile Pro
Leu Met Lys Ala Ala Phe Lys Arg Ser Cys Leu Lys His His
Pro Asp Lys Gly Gly Asn Pro Val Ile Met Met Glu Leu Asn
Thr Leu Trp Ser Lys Phe Gln Gln Asn Ile His Lys Leu Arg
Ser Asp Phe Ser Met Phe Asp Glu Val Asp Glu Ala Pro Ile
Tyr Gly Thr Thr Lys Phe Lys Glu Trp Trp Arg Ser Gly Gly
Phe Ser Phe Gly Lys Ala Tyr Glu Tyr Gly Pro Asn Pro His
Gly Thr Asn Ser Arg Ser Arg Lys Pro Ser Ser Asn Ala Ser
Arg Gly Ala Pro Ser Gly Ser Ser Pro Pro His Ser Gln Ser
Ser Ser Ser Gly Tyr Gly Ser Phe Ser Ala Ser Gln Ala Ser
Asp Ser Gln Ser Arg Gly Pro Asp Ile Pro Pro Glu His His
Glu Glu Pro Thr Ser Ser Ser Gly Ser Ser Ser Arg Glu Glu
Thr Thr Asn Ser Gly Arg Glu Ser Ser Thr Pro Asn Gly Thr
Ser Val Pro Arg Asn Ser Ser Arg Thr Asp Gly Thr Trp Glu
Asp Leu Phe Cys Asp Glu Ser Leu Ser Ser Pro Glu Pro Pro
Ser Ser Ser Glu Glu Pro Glu Glu Pro Pro Ser Ser Arg Ser
Ser Pro Arg Gln Pro Pro Ser Ser Ser Ala Glu Glu Ala Ser
Ser Ser Gln Phe Thr Asp Glu Glu Tyr Arg Ser Ser Ser Phe
Thr Thr Pro Lys Thr Pro Pro Pro Phe Ser Arg Lys Arg Lys
Phe Gly Gly Ser Arg Ser Ser Ala Ser Ser Ala Ser Ser Ala
Ser Phe Thr Ser Thr Pro Pro Lys Pro Lys Lys Asn Arg Glu
Thr Pro Val Pro Thr Asp Phe Pro Ile Asp Leu Ser Asp Tyr
Leu Ser His Ala Val Tyr Ser Asn Lys Thr Val Ser Cys Phe
Ala Ile Tyr Thr Thr Ser Asp Lys Ala Ile Glu Leu Tyr Asp
Lys Ile Glu Lys Phe Lys Val Asp Phe Lys Ser Arg His Ala
Cys Glu Leu Gly Cys Ile Leu Leu Phe Ile Thr Leu Ser Lys
His Arg Val Ser Ala Ile Lys Asn Phe Cys Ser Thr Phe Cys
Thr Ile Ser Phe Leu Ile Cys Lys Gly Val Asn Lys Met Pro
Glu Met Tyr Asn Asn Leu Cys Lys Pro Pro Tyr Lys Leu Leu
Gln Glu Asn Lys Pro Leu Leu Asn Tyr Glu Phe Gln Glu Lys
Glu Lys Glu Ala Ser Cys Asn Trp Asn Leu Val Ala Glu Phe
Ala Cys Glu Tyr Glu Leu Asp Glu Val Arg Asp Ala Gly Asn
Asp Gln Asp Arg Asn Ala Met
 1-letter code: 
MDLVLNRKEREALCKLLEIAPNCYGNIPLMKAAFKRSCLKHHPDKGGNPVIMMELNT
LWSKFQQNIHKLRSDFSMFDEVDEAPIYGTTKFKEWWRSGGFSFGKAYEYGPNPHGT
NSRSRKPSSNASRGAPSGSSPPHSQSSSSGYGSFSASQASDSQSRGPDIPPEHHEEP
TSSSGSSSREETTNSGRESSTPNGTSVPRNSSRTDGTWEDLFCDESLSSPEPPSSSE
EPEEPPSSRSSPRQPPSSSAEEASSSQFTDEEYRSSSFTTPKTPPPFSRKRKFGGSR
SSASSASSASFTSTPPKPKKNRETPVPTDFPIDLSDYLSHAVYSNKTVSCFAIYTTS
DKAIELYDKIEKFKVDFKSRHACELGCILLFITLSKHRVSAIKNFCSTFCTISFLIC
KGVNKMPEMYNNLCKPPYKLLQENKPLLNYEFQEKEKEASCNWNLVAEFACEYELDE
VRDAGNDQDRNAM
**SEQ ID NO : 2,** N-terminal end of ExoS from position 1 (including the Met N-ter) to position 129
   3-letter code : 
Met His Ile Gln Ser Leu Gln Gln Ser Pro Ser Phe Ala Val
Glu Leu His Gln Ala Ala Ser Gly Arg Leu Gly Gln IleGlu
Ala Arg Gln Val Ala Thr Pro Ser Glu Ala Gln Gln Leu Ala
Gln Arg Gln Asp Ala Pro Lys Gly Glu Gly Leu Leu Ala Arg
Leu Gly Ala Ala Leu Val Arg Pro Phe Val Ala Ile Met Asp
Trp Leu Gly Lys Leu Leu Gly Ser His Ala Arg Thr Gly Pro
Gln Pro Ser Gln Asp Ala Gln Pro Ala Val Met Ser Ser Ala
Val Val Phe Lys Gln Met Val Leu Gln Gln Ala Leu Pro Met
Thr Leu Lys Gly Leu Asp Lys Ala Ser Glu Leu Ala Thr Leu
Thr Pro Glu
 1-letter code: 
MHIQSLQQSPSFAVELHQAASGRLGQIEARQVATPSEAQQLAQRQDAPKGEGLLARLGAALVRPFVAI
MDWLGKLLGSHARTGPQPSQDAQPAVMSSAVVFKQMVLQQALPMTLKGLDKASELATLTPE
**SEQ ID NO : 3,** N-terminal end of ExoT, position 1 (including the Met N-ter) to position 129
   3-letter code: 
Met His Ile Gln Ser Ser Gln Gln Asn Pro Ser Phe Val Ala
Glu Leu Ser Gln Ala Val Ala Gly Arg Leu Gly Gln Val Glu
Ala Arg Gln Val Ala Thr Pro Arg Glu Ala Gln Gln Leu Ala
Gln Arg Gln Glu Ala Pro Lys Gly Glu Gly Leu Leu Ser Arg
Leu Gly Ala Ala Leu Ala Arg Pro Phe Val Ala Ile Ile Glu
Trp Leu Gly Lys Leu Leu Gly Ser Arg Ala His Ala Ser Thr
Gln Ala Pro Leu Ser Arg Gln Asp Ala Pro Pro Ala Ala Ser
Leu Ser Ala Ala Glu Ile Lys Gln Met Met Leu Gln Lys Ala
Leu Pro Leu Thr Leu Gly Gly Leu Gly Lys Ala Ser Glu Leu
Ala Thr Leu
 1-letter code : 
MHIQSSQQNPSFVAELSQAVAGRLGQVEARQVATPREAQQLAQRQEAPKGEGLLSRLGAALARPFVAI
IEWLGKLLGSRAHASTQAPLSRQDAPPAASLSAAEIKQMMLQKALPLTLGGLGKASELATL
**SEQ ID NO : 4,** PADRE sequence
   3-letter code:
   Ala Lys Phe Val Ala Ala Trp Thr Leu Lys Ala Ala Ala
   1-letter code :
   AKFVAAWTLKAAA
**SEQ ID NO : 5,** NP68 peptide (362-378)
   3-letter code: 
Gly Val Gln Ile Ala Ser Asn Glu Asn Met Asp Ala Met Glu
Ser Ser Thr
 1-letter code :
   GVQIASNENMDAMESST
**SEQ ID NO : 6,** OVA peptide (257-264)
   1-letter code : SIINFEKL
**SEQ ID NO : 7,** nucleotide sequence encoding the amino acid sequence from position 2 to 259 of SEQ ID NO : 1 which has been further mutated to position 216 (E216K), for optimized expression in *Pseudomonas* 
GACCTGGTTTTGAATCGCAAGGAACGTGAAGCATTGTGTAAGCTTCTGGAGATTGCC
CCGAACTGCTACGGAATATCCCGCTGATGAAAGCCGCTTTCAAGCGCTCGTGCCTG
AAGCACCACCCGGATAAGGGGGCAACCCGGTCATTATGATGGAGCTGAACACGCTT
TGGTCGAAGTTCCAACAGAACATCCACAAGCTGCGTTCGGACTTCTCCATGTTCGAT
GAGGTGGACGAAGCCCCGATCTACGGCACTACCAAGTTCAAAGAGTGGTGGCGCTCG
GGCGGCTTCTCGTTCGGAAAGGCATACGAGTATGGCCGAACCCTCATGGCACAAAC
AGCCGTTCGCGCAAACCGAGTAGTAACGCTTCGCGCGGTGCCCCGAGCGGAAGCTCG
CCGCCACATAGTCAGAGCTCGTCCAGCGGGTATGGCTCGTTTAGTGCAAGCCAGGCA
AGTGATTCCCAGAGTCGGGGCCCGGATATACCGCCCGAACACCACGAGGAGCCCACG
TCCTCCAGCGGTAGCTCGAGTCGGGAAGAAACGACCAACAGCGGACGCGAATCGAGC
ACGCCCAATGGCACCTCGGTTCCCCGCAATTCCTCGCGCACCGATGGTACTTGGGAA
GACTTGTTTTGCGACGAGAGTTTGTCCTCCCCGGAGCCTCCGAGCAGTAGCGAGGAA
CCGGAAGAGCCACCCTCGTCCCGTTCGAGTCCGCGGCAACCTCCGTCCTCCTCGGCA
GAGGAAGCAAGTAGCTCGCAATTCACCGACGAG
**SEQ ID NO : 8,** nucleotide sequence encoding the amino acid sequence of SEQ ID NO : 2 (ExoS), from position 1 to position 129 
ATGCATATTCAATCGCTTCAGCAGAGTCCGTCTTTCGCCGTCGAATTGCACCAGGCCGCCAGTGGGC
GTTTGGGACAGATTGAGGCCCGCCAGGTCGCCACCCCCAGTGAAGCGCAGCAGTTGGCCCAGCGC
 
CAGGACGCGCCGAAGGGTGAGGGGCTGCTCGCTCGCCTGGGCGCGGCGCTCGTGCGTCCCTTCGT
GGCGATCATGGACTGGCTGGGCAAACTGTTGGGCTCCCACGCCCGCACCGGCCCGCAGCCCAGTCA
GGACGCGCAGCCTGCGGTCATGTCCTCGGCCGTCGTGTTCAAGCAGATGGTGCTGCAGCAGGCATT
GCCCATGACCTTGAAGGGACTCGACAAGGCGAGCGAGCTGGCGACCCTGACACCGGAA
**SEQ ID NO : 9,** nucleotide sequence encoding the amino acid sequence of SEQ ID NO : 4 (PADRE sequence)
   GCCAAGTTCGTCGCCGCCTGGACCCTGAAGGCCGCCGCC
**SEQ ID NO : 10,** nucleotide sequence encoding the amino acid sequence of SEQ ID NO : 5 (peptide NP68) for optimized expression in *Pseudomonas*
   GGCGTGCAGATCGCCTCCAACGAAAATATGGACGCGATGGAGTCGAGCACC
**SEQ ID NO : 11,** PCR primer
   ACTTGGGAAAGTTTTGACTGGTGGCAA
**SEQ ID NO : 12,** PCR antisense primer
   GGGCCTCGTCAACCTAGATGGGAAAG
**SEQ ID NO : 13,** Native LT from position 1 to 817 
MDLVLNRKEREALCKLLEIAPNCYGNIPLMKAAFKRSCLKHHPDKGGNPVIMMELNT
LWSKFQQNIHKLRSDFSMFDEVDEAPIYGTTKFKEWWRSGGFSFGKAYEYGPNPHGT
NSRSRKPSSNASRGAPSGSSPPHSQSSSSGYGSFSASQASDSQSRGPDIPPEHHEEP
TSSSGSSSREETTNSGRESSTPNGTSVPRNSSRTDGTWEDLFCDESLSSPEPPSSSE
EPEEPPSSRSSPRQPPSSSAEEASSSQFTDEEYRSSSFTTPKTPPPFSRKRKFGGSR
SSASSASSASFTSTPPKPKKNRETPVPTDFPIDLSDYLSHAVYSNKTVSCFAIYTTS
DKAIELYDKIEKFKVDFKSRHACELGCILLFITLSKHRVSAIKNFCSTFCTISFLIC
KGVNKMPEMYNNLCKPPYKLLQENKPLLNYEFQEKEKEASCNWNLVAEFACEYELDD
HFIILAHYLDFAKPFPCQKCENRSRLKPHKAHEAHHSNAKLFYESKSQKTICQQAAD
TVLAKRRLEMLEMTRTEMLCKKFKKHLERLRDLDTIDLLYYMGGVAWYCCLFEEFEK
KLQKIIQLLTENIPKYRNIWFKGPINSGKTSFAAALIDLLEGKALNINCPSDKLPFE
LGCALDKFMVVFEDVKGQNSLNKDLQPGQGINNLDNLRDHLDGAVAVSLEKKHVNKK
HQIFPPCIVTANDYFIPKTLIARFSYTLHFSPKANLRDSLDQNMEIRKRRILQSGTT
LLLCLIWCLPDTTFKPCLQEEIKNWKQILQSEISYGKFCQMIENVEAGQDPLLNILI
EEEGPEETEETQDSGTFSQ
**SEQ ID NO : 14,** nucleotide sequence encoding the amino acid sequence from position 2 to 817 of SEQ ID NO: 13 for optimized expression in *Pseudomonas* 
GACCTGGTTTTGAATCGCAAGGAACGTGAAGCATTGTGTAAGCTTCTGGAGATTGCC
CCGAACTGCTACGGGAATATCCCGCTGATGAAAGCCGCTTTCAAGCGCTCGTGCCTG
AAGCACCACCCGGATAAGGGGGGCAACCCGGTCATTATGATGGAGCTGAACACGCTT
TGGTCGAAGTTCCAACAGAACATCCACAAGCTGCGTTCGGACTTCTCCATGTTCGAT
GAGGTGGACGAAGCCCCGATCTACGGCACTACCAAGTTCAAAGAGTGGTGGCGCTCG
 
GGCGGCTTCTCGTTCGGAAAGGCATACGAGTATGGCCGAACCCTCATGGCACAAAC
AGCCGTTCGCGCAAACCGAGTAGTAACGCTTCGCGCGGTGCCCCGAGCGGAAGCTCG
CCGCCACATAGTCAGAGCTCGTCCAGCGGTATGGCTCGTTTAGTGCAAGCCAGGCA
AGTGATTCCCAGAGTCGGGGCCCGGATATACCGCCCGAACACCACGAGGAGCCCACG
TCCTCCAGCGGTAGCTCGAGTCGGGAAGAAACGACCAACAGCGGACGCGAATCGAGC
ACGCCCAATGGCACCTCGGTTCCCCGCAATTCCTCGCGCACCGATGGTACTTGGGAA
GACTTGTTTTGCGACGAGAGTTTGTCCTCCCCGGAGCCTCCGAGCAGTAGCGAGGAA
CCGGAAGAGCCACCCTCGTCCCGTTCGAGTCCGCGGCAACCTCCGTCCTCCTCGGCA
GAGGAAGCAAGTAGCTCGCAATTCACCGACGAGGAGTATCGCTCCAGCAGCTTTACC
ACCCCTAAAACCCCGCCACCCTTCAGTCGCAAACGCAAGTTCGGTGGTTCGCGGTCC
AGCGCCAGTTCCGCCAGCTCCGCTAGCTTTACATCCACTCCTCCAAAGCCTAAGAAG
AACCGTGAAACCCCCGTCCCCACCGACTTCCCGATTGACCTGAGCGATTATCTGAGC
CATGCCGTTTACTCGAACAAAACCGTGAGCTGCTTCGCCATCTACACTACCAGCGAC
AAGGCCATCGAGTTGTATGACAAGATCGAGAAGTTCAAGGTGGACTTCAAAAGCCGG
CACGCCTGCGAGCTCGGCTGCATCCTTCTGTTCATCACACTGAGCAAACACCGCGTA
TCGGCGATCAAAAACTTCTGCAGCACGTTCTGCACGATCAGCTTCCTCATCTGCAAG
GGCGTGAACAAGATGCCGGAGATGTATAACAACCTGTGCAAACCGCCGTACAAGCTC
CTGCAAGAGAATAAGCCGCTCCTTAACTATGAGTTTCAGGAGAAGGAGAAAGAGGCG
TCCTGCAACTGGAACCTGGTGGCGGAGTTTGCGTGCGAGTATGAACTCGACGACCAC
TTCATTATCCTCGCCCACTACCTCGATTTTGCTAAGCCTTTCCCGTGTCAAAAGTGC
GAGAATCGGAGCCGTCTGAAGCCTCACAAAGCGCATGAGGCCCATCATTCGAACGCG
AAACTGTTCTACGAATCCAAGAGCCAGAAGACGATTTGCCAACAGGCGGCCGACACG
GTCCTTGCCAAACGTCGCCTGGAAATGTTGGAGATGACACGGACCGAGATGCTGTGC
AAGAAGTTTAAGAAACACCTGGAACGCCTCCGCGACTTGGATACTATTGATTTGCTC
TACTACATGGGCGGCGTAGCGTGGTACTGTTGCCTGTTCGAGGAGTTCGAGAAGAAG
CTCCAGAAAATCATACAGCTGCTCACAGAGAACATACCGAAATACCGCAACATCTGG
TTTAAGGGCCCGATCAATTCCGGCAAGACCAGCTTTGCGGCGGCGCTGATCGACCTC
TTGGAAGGCAAGGCGCTTAATATCAACTGTCCAAGCGATAAGCTGCCCTTCGAGCTG
GGTTGCGCCCTGGATAAATTCATGGTGGTTTTCGAAGATGTGAAGGGCCAGAACAGC
CTGAATAAGGACCTGCAGCCCGGGCAGGGCATTAACAATTTGGACAACCTGCGGGAT
CATCTTGACGGTGCTGTGGCGGTCAGCCTCGAGAAAAAGCATGTCAACAAGAAGCAT
CAAATCTTCCCGCCCTGCATCGTCACCGCCAATGATTACTTTATCCCGAAAACCCTG
ATCGCCCGCTTCTCCTATACGCTTCACTTCTCCCCAAAGGCTAATCTGCGCGACAGC
CTGGACCAGAATATGGAGATTCGCAAGCGCCGGATACTGCAGTCGGGGACCACCTTG
TTGCTGTGCCTGATCTGGTGTCTGCCCGACACCACCTTCAAACCATGTCTGCAGGAA
GAGATTAAGAATTGGAAGCAGATCCTCCAGTCGGAAATCAGCTACGGCAAGTTCTGT
CAGATGATTGAAAACGTCGAGGCCGGCCAGGACCCGCTGCTCAACATCCTGATCGAG
GAAGAAGGACCCGAAGAAACCGAAGAGACCCAGGATTCGGGCACGTTTAGCCAG
**SEQ ID NO : 15,** Native sT from position 1 to 186 
MDLVLNRKEREALCKLLEIAPNCYGNIPLMKAAFKRSCLKHHPDKGGNPVIMMELNT
LWSKFQQNIHKLRSDFSMFDEVSTKFPWEEYGTLKDYMQSGYNARFCRGPGCMLKQL
RDSKCACISCKLSRQHCSLKTLKQKNCLTWGECFCYQCFILWFGFPPTWESFDWWQK
TLEETDYCLLHLHLF
**SEQ ID NO : 16,** nucleotide sequence encoding the amino acid sequence from position 2 to 186 of SEQ ID NO : 15 for optimized expression in *Pseudomonas* 
GACCTGGTTTTGAATCGCAAGGAACGTGAAGCATTGTGTAAGCTTCTGGAGATTGCC
CCGAACTGCTACGGGAATATCCCGCTGATGAAAGCCGCTTTCAAGCGCTCGTGCCTG
AAGCACCACCCGGATAAGGGGGCAACCCGGTCATTATGATGGAGCTGAACACGCTT
TGGTCGAAGTTCCAACAGAACATCCACAAGCTGCGTTCGGACTTCTCCATGTTCGAT
GAGGTGTCCACCAAGTTTCCCTGGGAAGAGTACGGCACACTGAAGGATTACATGCAA
TCCGGATACAATGCGCGGTTTTGCCGCGGCCCTGGTTGTATGCTGAAACAGCTGCGC
GACAGCAAATGCGCCTGCATCAGTTGCAAACTGAGCCGGCAGCATTGCAGCCTCAAA
ACCCTCAAGCAGAAGAACTGCTTGACCTGGGCGAATGCTTCTGTTATCAGTGCTTC
ATCCTCTGGTTCGGCTTCCCCCCAACTTGGGAGAGCTTCGACTGGTGGCAGAAGACG
CTCGAGGAGACCGACTATTGTTTGCTCCATCTGCATCTGTTT
**SEQ ID NO** : **17,** nucleotide sequence encoding the amino acid sequence from position 81 to 186 of SEQ ID NO : 15 for optimized expression in 
ACCAAGTTTCCCTGGGAAGAGTACGGCACACTGAAGGATTACATGCAATCCGGATAC
AATGCGCGGTTTTGCCGCGGCCCTGGTTGTATGCTGAAACAGCTGCGCGACAGCAAA
TGCGCCTGCATCAGTTGCAAACTGAGCCGGCAGCATTGCAGCCTCAAAACCCTCAAG
CAGAAGAACTGCTTGACCTGGGGCGAATGCTTCTGTTATCAGTGCTTCATCCTCTGG
TTCGGCTTCCCCCCAACTTGGAGAGCTTCGACTGGTGGCAGAAGACGCTCGAGGAG
ACCGACTATTGTTTGCTCCATCTGCATCTGTTT
**SEQ ID NO** : **18,** nucleotide sequence encoding the amino acid sequence from position 2 to 141 of SEQ ID NO : 15 for optimized expression in *Pseudomonas* 
GACCTGGTTTTGAATCGCAAGGAACGTGAAGCATTGTGTAAGCTTCTGGAGATTGCC
CCGAACTGCTACGGGAATATCCCGCTGATGAAAGCCGCTTTCAAGCGCTCGTGCCTG
AAGCACCACCCGGATAAGGGGGCAACCCGGTCATTATGATGGAGCTGAACACGCTT
TGGTCGAAGTTCCAACAGAACATCCACAAGCTGCGTTCGGACTTCTCCATGTTCGAT
GAGGTGTCCACCAAGTTTCCCTGGAAGAGTACGGCACACTGAAGGATTACATGCAA
TCCGGATACAATGCGCGGTTTTGCCGCGGCCCTGGTTGTATGCTGAAACAGCTGCGC
GACAGCAAATGCGCCTGCATCAGTTGCAAACTGAGCCGGCAGCATTGCAGCCTCAAA
ACCCTCAAGCAGAAGAACTGC
**SEQ ID NO : 19,** nucleotide sequence encoding the amino acid sequence from position 81 to 141 of SEQ ID NO : 15 for optimized expression in *Pseudomonas* 
ACCAAGTTTCCCTGGGAAGAGTACGGCACACTGAAGGATTACATGCAATCCGGATAC
AATGCGCGGTTTTGCCGCGGCCCTGGTTGTATGCTGAAACAGCTGCGCGACAGCAAA
TGCGCCTGCATCAGTTGCAAACTGAGCCGGCAGCATTGCAGCCTCAAAACCCTCAAG
CAGAAGAACTGC
**SEQ ID NO : 20,** nucleotide sequence encoding the amino acid sequence of SEQ ID NO : 3 (ExoT), from position 1 to position 129 
ATGCATATTCAATCATCTCAGCAGAACCCGTCTTTCGTGGCTGAGTTGAGCCAGGCC
GTGGCCGGGCGCCTGGACAGGTCGAGGCCCGCCAGGTGGCCACTCCCCGGAGGCG
CAACAACTGGCCCAGCGCCAGGAAGCACCGAAGGGCGAGGGCCTGCTCTCCCGCCTG
GGGGCCGCCCTCGCGCGTCCCTTCGTGGCGATCATCGAGTGGCTGGGCAAACTGCTG
GGGAGCCGTGCCCACGCCTCCACCCAGGCGCCGCTCTCCCGTCAGGACGCGCCGCCT
GCCGCCAGTCTCTCGGCCGCCGAGATCAAGCAGATGATGCTGCAAAAGGCACTGCCC
CTGACCTTGGGCGGACTTGGCAAGGCGAGCGAGCTGGCGACTTTG
**SEQ ID NO : 21** nucleotide sequence encoding for the green fluorescent protein 
ATGGTGAGCAAGGGCGAGGAGCTGTTCACCGGGGTGGTGCCCATCCTGGTCGAGCTG
GACGGCGACGTAAACGGCCACAAGTTCAGCGTGTCCGGCGAGGGCGAGGGCGATGCC
ACCTACGGCAAGCTGACCCTGAAGTTCATCTGCACCACCGGCAAGCTGCCCGTGCCC
TGGCCCACCCTCGTGACCACCCTGACCTACGGCGTGCAGTGCTTCAGCCGCTACCCC
GACCACATGAAGCAGCACGACTTCTTCAAGTCCGCCATGCCCGAAGGCTACGTCCAG
GAGCGCACCATCTTCTTCAAGGACGACGGCAACTACAAGACCCGCGCCGAGGTGAAG
TTCGAGGGCGACACCCTGGTGAACCGCATCGAGCTGAAGGGCATCGACTTCAAGGAG
GACGGCAACATCCTGGGGCACAAGCTGGAGTACAACTACAACAGCCACAACGTCTAT
ATCATGGCCGACAAGCAGAAGAACGGCATCAAGGTGAACTTCAAGATCCGCCACAAC
ATCGAGGACGGCAGCGTGCAGCTCGCCGACCACTACCAGCAGAACACCCCCATCGGC
GACGGCCCCGTGCTGCTGCCCGACAACCACTACCTGAGCACCCAGTCCGCCCTGAGC
AAAGACCCCAACGAGAAGCGCGATCACATGGTCCTGCTGGAGTTCGTGACCGCCGCC
GGGATCACTCTCGGCATGGACGAGCTGTACAAG
**SEQ ID NO** : **22,** nucleotide sequence encoding the amino acid sequence from position 2 to 215 of SEQ ID NO : 1 for optimized expression in Pseudomonas 
GACCTGGTTTTGAATCGCAAGGAACGTGAAGCATTGTGTAAGCTTCTGGAGATTGCC
CCGAACTGCTACGGGAATATCCCGCTGATGAAAGCCGCTTTCAAGCGCTCGTGCCTG
AAGCACCACCCGGATAAGGGGGCAACCCGGTCATTATGATGGAGCTGAACACGCTT
TGGTCGAAGTTCCAACAGAACATCCACAAGCTGCGTTCGGACTTCTCCATGTTCGAT
GAGGTGGACGAAGCCCCGATCTACGGCACTACCAAGTTCAAAGAGTGGTGGCGCTCG
GGCGGCTTCTCGTTCGGAAAGGCATACGAGTATGGCCGAACCCTCATGGCACAAAC
AGCCGTTCGCGCAAACCGAGTAGTAACGCTTCGCGCGGTGCCCCGAGCGGAAGCTCG
CCGCCACATAGTCAGAGCTCGTCCAGCGGTATGGCTCGTTTAGTGCAAGCCAGGCA
AGTGATTCCCAGAGTCGGGGCCCGGATATACCGCCCGAACACCACGAGGAGCCCACG
TCCTCCAGCGGTAGCTCGAGTCGGGAAGAAACGACCAACAGCGGACGCGAATCGAGC
ACGCCCAATGGCACCTCGGTTCCCCGCAATTCCTCGCGCACCGATGGTACTTGGGAA
GACTTGTTTTGCGAC
**SEQ ID NO : 23** nucleotide sequence encoding the amino acid sequence from position 1 to 259 of SEQ ID NO : 1 which has been further mutated to position 216 (E216K) 
ATGGACCTTGTCCTTAACAGGAAGGAGCGTGAGGCTTTGTGCAAGCTGCTGGAAATC
GCTCCAAACTGCTACGGTAACATCCCTCTGATGAAGGCTGCCTTCAAGCGAAGCTGT
 
CTCAAGCACCATCCTGACAAGGGTGGAAACCCTGTGATCATGATGGAGCTCAACACC
CTGTGGTCAAAATTCCAGCAAAACATTCACAAGCTCCGATCCGACTTCTCCATGTTT
GACGAGGTTGACGAAGCCCCTATATACGGACCACCAAGTTTAAAGAGTGGTGGAGG
TCCGGAGGATTCAGCTTTGGGAAAGCATACGAGTATGGGCCCAACCCCCACGGAACC
AATTCCCGGTCCCGGAAACCTTCCTCCAATGCTTCCAGGGGCGCACCAAGCGGTTCT
TCCCCTCCACATTCTCAATCTTCCAGCTCCGGTTATGGCTCTTTCTCCGCATCACAG
GCCTCAGATTCTCAGAGCAGAGGCCCAGATATTCCTCCAGAACATCACGAGGAGCCA
ACTTCTAGTAGCGGGTCAAGTAGTAGAGAGGAGACTACCAATAGCGGCAGAGAGAGC
AGCACACCAAATGGCACAAGCGTACCTCGCAATAGCAGCCGCACAGATGGCACATGG
GAAGATTTGTTTTGTGACAAATCACTGTCTTCTCCTGAGCCCCCAGTTCTTCTGAG
GAACCCGAAGAACCCCCATCATCACGCAGTAGTCCCCGGCAGCCCCCCTCTTCAAGT
GCCGAAGAAGCCAGTAGTTCTCAGTTTACTGAT
**SEQ ID NO : 24** Nucleotide sequence encoding an IRES 
CCCCCCCCCCCTAACGTTACTGGCCGAAGCCGCTTGGAATAAGGCCGGTGTGCGTTT
GTCTATATGTTATTTTCCACCATATTGCCGTCTTTTGGCAATGTGAGGGCCCGGAAA
CCTGGCCCTGTCTTCTTGACGAGCATTCCTAGGGGTCTTTCCCCTCTCGCCAAAGGA
ATGCAAGGTCTGTTGAATGTCGTGAAGGAAGCAGTTCCTCTGGAAGCTTCTTGAAGA
CAAACAACGTCTGTAGCGACCCTTTGCAGGCAGCGGAACCCCCCACCTGGCGACAGG
TGCCTCTGCGGCCAAAAGCCACGTGTATAAGATACACCTGCAAAGGCGGCACAACCC
CAGTGCCACGTTGTGAGTTGGATAGTTGTGGAAAGAGTCAAATGGCTCTCCTCAAGC
GTATTCAACAAGGGGCTGAAGGATGCCCAGAAGGTACCCCATTGTATGGGATCTGAT
CTGGGGCCTCGGTGCACATGCTTTACATGTGTTTAGTCGAGGTTAAAAAACGTCTAG
GCCCCCCGAACCACGGGGACGTGGTTTTCCTTTGAAAAACACGATGATAAT
**SEQ ID NO : 25** nucleotide sequence of promoter EF1a 
CGTGAGGCTCCGATGCCCGTCAGTGGGCAGAGCGCACATCGCCCACAGTCCCCGAGA
AGTTGGGGGGAGGGGTCGGCAATTGAACCGGTGCCTAGAGAAGGTGGCGCGGGGTAA
ACTGGGAAAGTGATGTCGTGTACTGGCTCCGCCTTTTTCCCGAGGGTGGGGGAGAAC
CGTATATAAGTGCAGTAGTCGCCGTGAACGTTCTTTTTCGCAACGGGTTTGCCGCCA
GAACACA
**SEQ ID NO : 26** amino acid sequence of pRb

### Bibliography

1. Forbes NS, Nat. Rev. Cancer (2010) 10 : 785 Engineering the perfect (bacterial) cancer therapy*.*
2. Bachmann MF & Jennings GT, Nat. Rev. Immunol. (2010) 10 : 787 Vaccine delivery : a matter of size, geometry, kinetics and molecular patterns*.*
3. Banchereau J & Palucka AK, Nat. Rev. Immunol. (2005) 5 : 296 Dendritic cells as therapeutic vaccines against cancer*.*
4. Radics et al, Nature Structural & Mol. Biol. (2014) 21 (1) : 82 Structure of a pathogenic type 3 secretion system in action*.*
5. Chamekh M, Immunopharmacol. Immunotoxicol. (2010) 32 : 1. Immunomodulation using genetically engineered bacteria for type III-mediated delivery of heterologous antigens and cytokines : potential application in vaccine and therapeutical developments*.*
6. Tseng et al, BMC Biotech. (2009) 9 (suppl 1): 52 Protein secretion systems in bacterial-host associations*.*
7. Hegazy WA et al, Infect Immun. (2012) 80: 1193 Evaluation of Salmonella enterica type III secretion system effector proteins as carriers for heterologous vaccine antigens*.*
8. Spreng S et al, Methods (2006) 38 : 133 Rational design of Salmonella-based vaccination strategies*.*
9. Stocker BA. Vaccine (1988) 6 : 141 Auxotrophic Salmonella typhi as live vaccine*.*
10. VanCott JL et al, Nat. Med. (1998) 4 : 1247 Regulation of host immune responses by modification of Salmonella virulence genes*.*
11. Shuda et al, Proc. Nat. Acad. Sc. (2008) 105 (42): 16272 T antigen mutations are a human tumor-specific signature for Merkel cell polyomavirus*.*
12. Feng et al, Science (2008) 319 (5866): 1096 Clonal integration of a polyomavirus in human Merkel cell carcinoma*.*
13. Rodig et al, J Clin Invest. (2012) 122 (12) : 4645 Improved detection suggests all Merkel cell carcinomas harbor Merkel polyomavirus*.*
14. Cheng et al, J. Virol, (June 2013) 87 (11): 6118 Merkel cell polyoma large T antigen has growth-promoting and inhibitory activities*.*
15. Zeng et al, Vaccine (2012) 30 (7) : 1322 Development of a DNA vaccine targeting Merkel cell polyomavirus*.*
16. Gomez et al, Cell & Bioscience (2012) 2 : 36 Strategy for eliciting antigen-specific CD8+ T-cell mediated immune response against a cryptic CTL epitope of Merkel cell polyoma virus large T antigen*.*
17. Basu et al, J. Exp. Med. (1999) 189 (5): 797
18. UJF WO 2005/049644, EP 1 692 162 and US 7 939 319
19. UJF WO 2013/087667
20. Toussaint et al, Biochem. Biophys. Res. Commun. (1993) 196 : 416
21. Polack B et al, Biochem. Biophys. Res. Commun. (2000) 275 : 854 Protein delivery by Pseudomonas type III secretion system: Ex vivo complementation of p67(phox)-deficient chronic granulomatous disease*.*
22. Epaulard O et al, Mol. Ther. (2006) 14 : 656 Anti-tumor immunotherapy via antigen delivery from a live attenuated genetically engineered Pseudomonas aeruginosa type III secretion system-based vector*.*
23. Epaulard O et al, Clin. Vaccine Immunol. (2008) 15 : 308 Optimization of a type III secretion system-based Pseudomonas aeruginosa live vector for antigen delivery*.*
24. Le Gouëllec et al, Human vaccines & Immunotherapeutics (2012) 8 (10): 1454.
25. Le Gouëllec et al, Mol. Therapy (2013) 21 (5) : 1076
26. Quénée et al, BioTechniques (2005) 38 (1): 63
27. Wang Y et al, J. Immunother. (2012) 35 : 223 Optimization of antitumor immunotherapy mediated by type III secretion system-based live attenuated bacterial vectors*.*
28. Dubensky TW et al, Curr Opin Biotechnol. (2012) Killed but metabolically active vaccines*.*
29. Irsch J et al, Transfus. Med. Hemother. (2011) 38 : 19 Inactivation of Platelet and Plasma Blood Components for Transfusion Using the INTERCEPT Blood System™*.*
30. Brockstedt DG et al, Nat. Med. (2005) 11 : 853 Killed but metabolically active microbes: a new vaccine paradigm for eliciting effector T-cell responses and protective immunity*.*
31. Spreng S & Viret JF, Vaccine (2005) 23 : 2060 Plasmid maintenance systems suitable for GMO-based bacterial vaccines*.*
32. Curtiss R, 3rd et al, Res. Microbiol. (1990) 141 : 797 Stabilization of recombinant avirulent vaccine strains in vivo*.*
33. Andres et al, J. Cutan. Pathol. (2010) 37 : 28
34. Cimino et al, Exp. & Mol. Pathol. (2013) 94 : 40
35. DuThanh et al, J. Dermatol. Sc ; (2013) 71 : 138
36. Dworkin et al, J. Investigative Dermatol. (2009) 129 : 2868
37. Imajoh et al, Virology (2012) 9 : 154
38. Kassen et al, Int. J. Cancer (2009) 125 : 356
39. Kreuter et al, Arch. Dermatol. (2011) 147 (12) 1449
40. Murakami et al, J Clin. Virol. (2011) 50 : 37
41. Pantulu et al, Blood (2010) 116 (24): 5280
42. Scola et al, British J. Dermatology (2012) 167 : 1315
43. Hashida et al, Br. J. Cancer. (2013 Feb 19)108 (3): 629 Detection of Merkel cell polyomavirus with a tumour-specific signature in non-small cell lung cancer*.*
44. Antoniu et al, (2013) Int. J. Cancer (2013 Dec 15)133 (12) : 3016 Molecular pathological findings of Merkel cell polyomavirus in lung cancer: a possible etiopathogenetic link ?
45. Karia et al, (2013) J. Am. Acad. Dermatol. (2013 June) 68 (6): 957 Cutaneous squamous cell carcinoma: estimated incidence of disease, nodal metastasis, and deaths from disease in the United States, 2012*.*
46. Mamalaki et al, Dev. Immunol. (1993) 3 (3): 159 Positive and negative selection in transgenic mice expressing a T-cell receptor specific for influenza nucleoprotein and endogenous superantigen*.*
47. Hogquist et al, Cell (1994) 76 (1) : 17 T-cell receptor antagonist peptides induce positive selection*.*
48. Clarke et al, Immunol. Cell Biol. (2000) 78 : 110 Characterization of the ovalbumin-specific TCR transgenic line OT-I : MHC elements for positive and negative selection*.*
49. Xu et al, Lung Cancer (2014) 83 : 341
50. Megan et al, Virology (2013) 435 (1) : 118 Merel cell polyoma virus : A newly discovered human virus with oncogenic potential*.*
51. Showalter et al, Cell Host Microbe (2010) 7 (6) : 509 Merkel cell Polyoma virus and two Polyoma viruses are chronically shed from human skin*.*
52. D'Addario et al, Annals of Oncology (2010) 21 (Suppl 5) : 116
53. Lasithiotaki et al, Internat. J. Cancer (2013) 133 : 604
54. DHHS Pittburgh (Moore) WO 2009/079481 and US 8 524 248
55. U. Florida WO 2012/012605 and US 8 617 888
56. Institut Curie US 2011182901
57. Alexander et al, Immunity (1994) 1 (9) : 751
58. Needleman et al, J. Mol. Biol. (1970) 48 : 444
59. Sambrook et al., 2012, Molecular Cloning:A Laboratory Manual, Fourth Edition, Cold Spring Harbor Laboratory Press.
60. H.A Carleton et al. Nature communication Nature Communications 4, Article number: 1590 doi:10.1038/ncomms2594
61. Edgren T, Forsberg A, Rosqvist R, Wolf-Watz H. PLoS Pathog. 2012;8(5):e1002669. doi: 10.1371/journal.ppat.1002669. Epub 2012 May 10. Type III secretion in Yersinia: injectisome or not?
62. Le Gouëllec A, Chauchet X, Laurin D, Aspord C, Verove J, Wang Y, Genestet C, Trocme C, Ahmadi M, Martin 5, Broisat A, Cretin F, Ghezzi C, Polack B, Plumas J, Toussaint B. Mol Ther. 2013 May;21(5):1076-86. doi: 10.1038/mt.2013.41. Epub 2013 Mar 26.
   PMID: 23531551
   A safe bacterial microsyringe for in vivo antigen delivery and immunotherapy.
63. Krall R1, Zhang Y, Barbieri JT. J Biol Chem. 2004 Jan 23;279(4):2747-53. Epub 2003 Nov 3. Intracellular membrane localization of pseudomonas ExoS and Yersinia YopE in mammalian cells.

## Claims

1. A fusion protein which comprises from its N-terminal end to its C-terminal end and fused in frame:
- at least one secretion peptide signal able to direct said fusion protein to the type 3 secretion system of a bacterial vector, when said fusion protein is in a bacterial vector owning a type 3 secretion system, and
- one truncated form of the Large T (LT) antigen of the Merkel Cell Polyoma Virus (MCPγV), which has an amino acid sequence having at least 80 % identity with one of the amino acid sequences shown in SEQ ID NO : 1 which starts with the amino acid in any one of positions 1 to 5 and ends with the amino acid in any one of the positions 210 to 469,
and provided that said fusion protein does not bind to the human retinoblastoma protein.

2. The fusion protein according to claim 1, wherein the truncated form of the Large T antigen of the Merkel Cell Polyoma Virus has:
∘ an amino acid sequence having at least 80% identity with the amino acid sequence shown in SEQ ID NO : 1 which starts with the amino acid in position 2 and ends with the amino acid in position 215 and provided that said truncated form of the Large T antigen of the Merkel Cell Polyoma Virus does not bind to the human retinoblastoma protein, or
∘ an amino acid sequence having at least 80% identity with the amino acid sequence shown in SEQ ID NO : 1 which starts with the amino acid in position 2 and ends with the amino acid in position 270, and provided that said truncated form of the Large T antigen of the Merkel Cell Polyoma Virus does not bind to the human retinoblastoma protein, or
∘ one of the amino acid sequences shown in SEQ ID NO : 1 which starts with the amino acid in any one of positions 1 to 5, advantageously in position 2, and ends with the amino acid in any one of positions 210 to 215 and provided that said truncated form of the Large T antigen of the Merkel Cell Polyoma Virus does not bind to the retinoblastoma protein, or
∘ one of the amino acid sequences shown in SEQ ID NO : 1 which starts with the amino acid in any one of positions 1 to 5, advantageously in position 2, and ends with the amino acid in any one of positions 216 to 270, which is further mutated in the human retinoblastoma protein binding site located from position 212 to 216 in SEQ ID NO : 1 so that said truncated form of the Large T antigen of the Merkel Cell Polyoma Virus does not bind to the human retinoblastoma protein.

3. The fusion protein according to claim 1, wherein the truncated form of the Large T antigen of the Merkel Cell Polyoma Virus has one of amino acid sequences shown in SEQ ID NO : 1 which starts with the amino acid in any one of positions 1 to 5 , advantageously in position 2, and ends with the amino acid in position 215, and provided that said truncated form of the Large T antigen of the Merkel Cell Polyoma Virus does not bind to the human retinoblastoma protein.

4. A fusion protein according to claim 2, wherein the truncated form of the Large T antigen of the Merkel Cell Polyoma Virus has:
∘ one of the amino acid sequences shown in SEQ ID NO : 1 which starts with the amino acid in any one of positions 1 to 5, advantageously in position 2, and ends with the amino acid in any one of positions 250 to 260, advantageously in position 259, which is further mutated in the human retinoblastoma protein binding site located from position 212 to 216 in SEQ ID NO : 1, so that said truncated form of the Large T antigen of the Merkel Cell Polyoma Virus does not bind to the human retinoblastoma protein; or
∘ an amino acid sequence having at least 85 % identity with the amino acid sequence shown in SEQ ID NO : 1 which starts with the amino acid in position 2 and ends with the amino acid in position 259 and provided that said truncated form of the Large T antigen of the Merkel Cell Polyoma Virus does not bind to the human retinoblastoma protein.

5. The fusion protein according to claim 2 or 4, wherein the fusion protein is mutated in the human retinoblastoma protein binding site in position 216 in SEQ ID NO : 1.

6. The fusion protein according to claim 5, wherein the mutation in position 216 in SEQ ID NO : 1 is a substitution mutation replacing the Glu residue by a Lys residue (E216→K).

7. The fusion protein according to any one of the preceding claims, wherein the secretion peptide signal that is able to direct said fusion protein to the type 3 secretion system of a bacterial vector is the N-terminal moiety of the *Pseudomonas exo*S gene product and has one of the amino acid sequences shown in SEQ ID NO : 2 which starts with the amino acid in position 1 and ends with the amino acid in any one of positions 15 to 129, advantageously ends with the amino acid in any one of positions 15 to 70, advantageously ends with the amino acid in position 54.

8. The fusion protein according to any one of the preceding claims, wherein it further comprises a Pan-HLA-DR-binding epitope.

9. A bacterial vector owning a type 3 secretion system which is able to express, secrete and transfer, preferably translocate, into mammalian cells, the fusion protein as defined in any one of the claims 1 to 8.

10. The bacterial vector according to claim 9, wherein said bacterial vector is an attenuated bacterium.

11. The bacterial vector according to the claim 10, wherein the bacterium belongs to the genus of *Pseudomonas,* in particular wherein the bacterium belongs to the *Pseudomonas aeruginosa* species or to the *Pseudomonas syringuae species.*

12. The bacterial vector according to any one of the claims 10 to 11, which is unable to express at least one of the products chosen among the *exo*S, *exo*T, *exo*U and *exo*Y gene products and NDK cytotoxin, preferably is unable to express at least the *exo*S, exoT and exoU gene products.

13. The bacterial vector according to any one of the claims 10 to 12, which comprises an expression cassette, wherein the nucleotide sequence encoding the fusion protein as defined in any one of the claims 1 to 8 is placed under the control of the *exo*S promoter.

14. The bacterial vector according to any one of the claims 9 to 13, for use in a method of preventing or combating MCPγV infection, in particular by promoting a CD8+ immune response against MCPyV-infected cells.

15. The bacterial vector according to claim 14, wherein the CD8+ immune response is cytotoxic.

16. The bacterial vector according to claim 14 or 15, wherein the immune response is against cells (a) expressing whole or part of the MCPγV LT antigen and (b) in the genome of which is integrated an MCPγV nucleotide sequence encoding said whole or part of the LT antigen.

17. The bacterial vector according to any one of claims 14 to 16, wherein the method of combating or preventing MCPγV infection prevents the onset of a tumoral disorder in patients infected with MCPγV.

18. The bacterial vector according to any one of the claims 14 to 16, wherein the method of combating or preventing MCPγV infection reduces the growth or propagation of a tumor in a patient suffering from a tumoral disorder **characterized by** the presence of tumoral cells expressing the LT antigen of MCPγV.

19. The bacterial vector according to any one of the claims 9 to 13, for use as a medicament, preferably for use as a medicament in the treatment of cancer.
